(19) Europäisches Patentamt
European Patent Office
Office européen des brevets

(11) Veröffentlichungsnummer: **0 034 778**
**B1**

(12) # EUROPÄISCHE PATENTSCHRIFT

(45) Veröffentlichungstag der Patentschrift: **25.04.84**

(21) Anmeldenummer: **81101037.0**

(22) Anmeldetag: **14.02.81**

(51) Int. Cl.³: **C 07 D 209/52**, C 07 D 409/12, A 61 K 31/40 // C07D307/93, C07D309/12, C07D405/12

(54) **Hetero-Imino-Prostacycline und Verfahren zu ihrer Herstellung.**

(30) Priorität: **23.02.80 DE 3006865**

(43) Veröffentlichungstag der Anmeldung: **02.09.81 Patentblatt 81/35**

(45) Bekanntmachung des Hinweises auf die Patenterteilung: **25.04.84 Patentblatt 84/17**

(84) Benannte Vertragsstaaten: **AT BE CH DE FR GB IT LI NL SE**

(56) Entgegenhaltungen:
**DE - A - 2 909 361**
**US - A - 4 097 489**

**ANGEWANDTE CHEMIE, Band 19, Nr. 10, Oktober 1980, Seiten 819-820 Weinheim, DE. H. KONIG: "Pharmaceutical chemistry today - Changes, problems, and opportunities"**
**Tetrahedron Lett. No. 16 (1978), S. 1371-74**
**Negwev, Organ.-Chem. Arzneimittel Berlin 1978, Nr. 6547, S. 1251**
**"Progress in Drug Research", Basel 1973, S. 469 (Vol. 17)**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Beck, Gerhard, Dr., Gustav-Freytag-Strasse 24, D-6000 Frankfurt am Main (DE)**
Erfinder: **Knolle, Jochen, Dr., Höchster Strasse 21, D-6239 Kriftel (DE)**
Erfinder: **Rupp, Richard Helmut, Dr., Röderweg 16a, D-6240 Königstein/Taunus (DE)**
Erfinder: **Schölkens, Bernward, Dr., Am Fliedergarten 1, D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 034 778 B1

BUNDESDRUCKEREI BERLIN

Hetero-Imino-Prostacycline und Verfahren zu ihrer Herstellung

Prostacyclin $PGI_2$, ein kürzlich isolierter Naturstoff aus der Familie der Prostaglandine, zeichnet sich durch seine stark ausgeprägten thrombocytenaggregationshemmenden Eigenschaften aus (The Lancet 1977, 18). Außerdem vermag $PGI_2$ einige Blutgefäße, z. B. Coronararterien zu relaxieren (Prostaglandins 13, 3, 1977), so daß es zur Therapie und Prophylaxe von Thrombosen und Infarkten Verwendung finden kann. $PGI_2$ zeigt weiter eine ausgeprägte blutdrucksenkende Wirkung (z. B. IRCS Med. Sci. 6, 392 [1978]).

Gegenstand der vorliegenden Erfindung sind neue Analoga des $PGI_2$ der allgemeinen Formel I

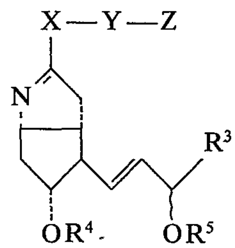

(I)

die eine spezifischere Wirkung und/oder eine längere Wirkungsdauer als $PGI_2$ besitzen und in welcher bedeuten:

X ein Sauerstoff- oder Schwefelatom oder eine NH-Gruppe,

Y einen geradkettigen oder verzweigten Alkylenrest mit bis zu acht Kohlenstoffatomen oder einen geradkettigen oder verzweigten ungesättigten aliphatischen Rest mit drei bis acht Kohlenstoffatomen oder einen cycloaliphatischen Rest mit drei bis sechs Kohlenstoffatomen oder einen Phenylenrest,

Z einen Rest der Formel $-CO_2R^1$, $-CH_2OH$ oder $CH_2-N(R^2)_2$ wobei bedeuten

$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit drei bis sechs Kohlenstoffatomen oder einen cycloaliphatischen Kohlenwasserstoffrest mit drei bis sieben Kohlenstoffatomen oder einen araliphatischen Kohlenwasserstoffrest mit sieben bis neun Kohlenstoffatomen, oder ein physiologisch verträgliches Metall-, $NH_4$- oder Ammoniumion wie nachstehend definiert

$R^2$ Wasserstoff oder einen geradkettigen oder verzweigten aliphatischen Kohlenwasserstoffrest bis zu fünf C-Atomen oder $R^2-R^2$ zusammen auch eine $-(CH_2)_n$-Gruppe, mit $n = 3-6$

$R^3$ unsubstituierten Phenylrest, oder einen Phenyl-, Thienyl- oder Furylrest der im Kern 1—3fach substituiert sein kann mit Halogen, Trifluormethyl, und/oder Alkyl oder Alkoxy mit je 1—6 C-Atomen, oder einen cycloaliphatischen Rest mit 3—8 Kohlenstoffatomen oder
einen geradkettigen oder verzweigten Alkylrest bis zu acht Kohlenstoffatomen oder einen geradkettigen oder verzweigten ungesättigten aliphatischen Kohlenwasserstoffrest mit drei bis acht Kohlenstoffatomen, die ihrerseits substituiert sein können mit

a) einem geradkettigen oder verzweigten Alkoxyrest mit bis zu sechs Kohlenstoffatomen oder einem geradkettigen oder verzweigten Alkenyloxy- oder Alkinyloxyrest mit drei bis sechs Kohlenstoffatomen,

b) Halogen, Phenyl oder einem $\alpha$- oder $\beta$-Thienyl- oder $\alpha$- oder $\beta$-Furylrest, die ihrerseits im Kern 1—3fach substituiert sein können mit Halogen, Trifluormethyl, und/oder Alkyl oder Alkoxy mit je 1—6 C-Atomen,

c) einem Phenoxy-, einem $\alpha$-oder $\beta$-Thienyloxyrest oder einem Cycloalkoxyrest mit 3—7 Kohlenstoffatomen, wobei die genannten Reste ihrerseits im Kern 1—3fach substituiert sein können mit Halogen, Trifluormethyl und/oder Alkyl oder Alkyloxy mit je 1—6 C-Atomen,

$R^4$, $R^5$ jeweils Wasserstoff oder eine, unter neutralen oder basischen Bedingungen leicht abspaltbare Schutzgruppe.

Unter den Substituenten $R^1$ sind genannt:

Wasserstoff, geradkettiges oder verzweigtes Alkyl mit bis zu 8 C-Atomen, ein geradkettiger oder verzweigter ungesättigter Kohlenwasserstoffrest mit bis zu 4 C-Atomen, ein cycloaliphatischer Kohlenwasserstoffrest mit 5—7 C-Atomen, ein araliphatischer Kohlenwasserstoffrest mit 8 oder 9 C-Atomen oder ein Ammoniumion wie Wasserstoff, Methyl, Äthyl, n-Butyl, n-Pentyl, n-Hexyl, n-Heptyl, 2-Propyl, 2-Butyl, 2-Pentyl, 3-Hexyl, 2-Methylpropyl, 2-Methylbutyl, 4,4-Dimethylpentyl, 5,5-Dimethyl-Dicyclohexylammonium, Tris(hydroxymethy)methylammonium.

Unter den Substituenten $R^3$ sind die im folgenden aufgeführten besonders bevorzugt:

unsubstituiertes Phenyl oder mit Halogen, Trifluormethyl, $C_1$—$C_4$-Alkyl oder $C_1$—$C_4$-Alkoxy einfach substituiertes Phenyl, geradkettiges oder verzweigtes $C_{3-7}$-Alkyl, das substituiert sein kann mit gegebenenfalls substituiertes $C_{5-7}$-Cycloalkyl, mit $C_{1-3}$-Alkoxy, mit Phenoxy oder Halogenphenoxy, mit Thienyloxy oder Halogenthienyloxy, mit Cyclohexyloxy, mit Thienyl, mit Halogenthienyl oder mit Furyl, insbesondere:

n-Pentyl, 1,1-Dimethylpentyl, Cyclopentylmethyl, Cyclohexylmethyl, 1,1-Dimethyl-2-äthoxy-äthyl, 1,1-Dimethyl-2-methoxy-äthyl, 1,1-Dimethyl-cyclohexyloxymethyl, 1-Fluorpentyl, 1-Chlorpentyl, 5-Fluorpentyl, 5-Chlorpentyl, 2-(Thien-3-yl)-äthyl, 2-(Thien-2-yl)-äthyl, 2-(2-Chlor-thien-3-yl)-äthyl, 2-(5-Chlor-thien-2-yl)-äthyl, Phenoxymethyl, 3-Chlorphenoxymethyl, Thien-2-yl-oxymethyl, 2-Chlor-thien-3-yl-oxymethyl, 5-Chlor-thien-2-yl-oxymethyl, 2-(Fur-3-yl)-äthyl, 2-(Fur-2-yl)-äthyl, 2-(2,2,3,3-tetrafluorcyclobutyl)-äthyl, Phenyl, 3-Chlor-phenyl, 3-Trisfluormethyl-phenyl.

Unter den Substituenten Y sind die im folgenden ausgeführten besonders bevorzugt:

Ethyliden, Trimethylen, Methyltrimethylen, Methyltetramethylen, Propenylen, Cyclobutenylen, Cyclopentenylen, Phenylen.

Gegenstand der Erfindung ist außerdem ein Verfahren zur Herstellung von Prostacyclinderivaten der Formel I, das dadurch gekennzeichnet ist, daß man

a) den Alkohol der Formel II

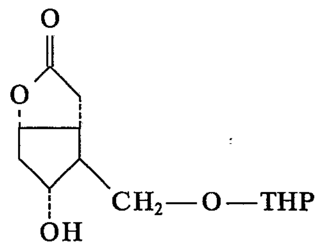

(II)

worin THP den Tetrahydropyranylrest bedeutet
zum Benzylether der Formel III umsetzt

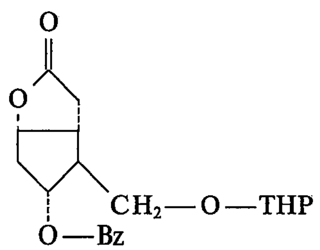

(III)

worin Bz den Benzylrest bedeutet,

b) das Lacton der Formel III mit Ammoniak zum Hydroxyamid der Formel IV öffnet

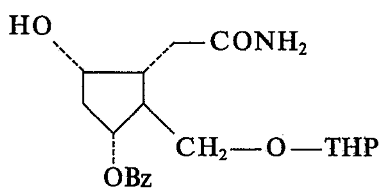

(IV)

c) die Hydroxyl-Funktion im Alkohol der Formel IV zum Keton der Formel V oxidiert

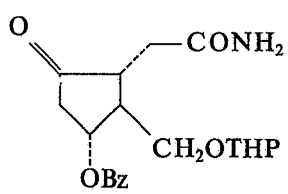

(V)

d) das Ketoamid der Formel V zum Hydroxylactam der Formel VI isomerisiert

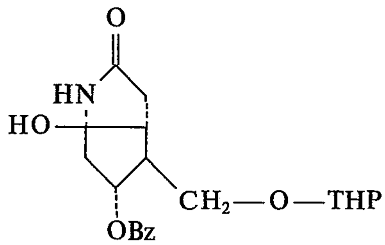

(VI)

e) das Hydroxylactam der Formel VI, das Ketoamid der Formel V oder eine Mischung aus Hydroxylactam der Formel VI und Ketoamid der Formel V mit einem Mercaptan der Formel VII

$R^6$—SH (VII)

in dem $R^6$ einen Alkylrest mit 1–5 C-Atomen oder einen Phenylrest bedeutet, umsetzt zu einem Thioether der Formel VIII

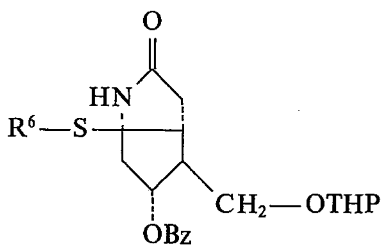

(VIII)

worin $R^6$ die zur Formel VII gegebenen Bedeutungen hat,

f) im Thioether der Formel VIII den Rest — $SR^6$ durch Reduktion abspaltet, wobei ein Lactam der Formel IX

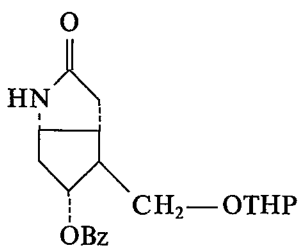

(IX)

erhalten wird,

g) die Benzylethergruppe im Lactam der Formel IX abhydriert, wobei ein Hydroxylactam der Formel X erhalten wird

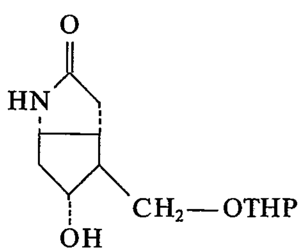

(X)

oder

g) die Abspaltung der Gruppe — $SR^6$ und der Benzylethergruppe in einem Schritt durchführt,

h) die Hydroxylfunktion im Lactam der Formel X durch eine Gruppe schützt, die unter neutralen oder basischen Bedingungen wieder abgespalten werden kann, wodurch ein Lactam der Formel XI

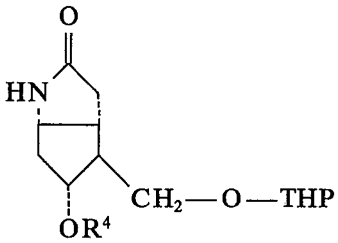

(XI)

erhalten wird,
worin $R^4$ eine unter neutralen oder basischen Bedingungen leicht abspaltbare Schutzgruppe bedeutet,

i) durch selektive Hydrolyse die THP-Gruppe im Lactam der Formel XI abspaltet, wobei ein Alkohol der Formel XII

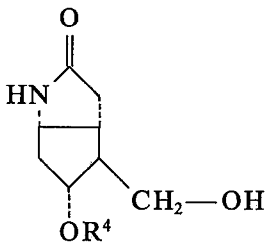

(XII)

erhalten wird,
worin $R^4$ die zur Formel XI gegebene Bedeutung hat

j) den Alkohol der Formel XII zum Aldehyd der Formel XIII oxidiert

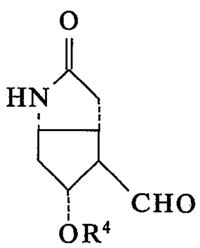

(XIII)

worin $R^4$ die zur Formel XI gegebene Bedeutung hat,

k) den Aldehyd der Formel XIII mit einem Phosphonat der Formel XIV

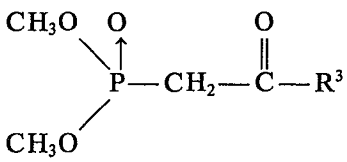

(XIV)

worin $R^3$ die zur Formel I gegebenen Bedeutungen hat, zu einem Enon der Formel XV

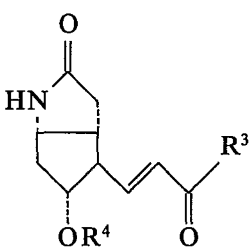

(XV)

worin $R^4$ die zur Formel XI und $R^3$ die zur Formel I gegebenen Bedeutungen haben, umsetzt,

l) das Enon der Formel XV in bekannter Weise mit einem geeigneten Reduktionsmittel zu einem Epimerengemisch der Alkohole der Formel XVI

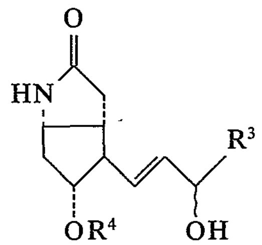

(XVI)

reduziert, worin $R^3$ die zur Formel I gegebenen Bedeutungen hat und $R^4$ die zur Formel XI gegebenen Bedeutungen hat,

m) die Alkoholfunktion in den Alkoholen der Formel XVI durch eine Gruppe schützt, die unter neutralen oder basischen Bedingungen wieder abgespalten werden kann, wodurch Verbindungen der Formel XVII

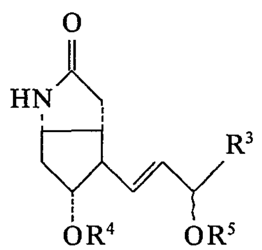

(XVII)

erhalten werden, in der $R^3$ die zur Formel I gegebenen Bedeutungen hat, $R^4$ die zur Formel XI gegebene Bedeutung hat und $R^5$ eine unter neutralen oder basischen Bedingungen leicht abspaltbare Schutzgruppe bedeutet, wobei $R^4$ und $R^5$ gleich oder verschieden sein können,

n) die Lactame der Formel XVII durch übliche Methoden in die Thiolactame der Formel XVIII überführt

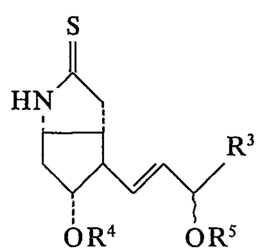

(XVIII)

worin $R^3$ die zur Formel I gegebene Bedeutung hat und $R^4$, $R^5$ die zur Formel XVII gegebene Bedeutung haben,

o) die Thiolactame der Formel XVIII mit Alkylhalogeniden der Formel IXX alkyliert

Z—Y—Hal (IXX)

worin Hal Jod, Chlor oder Brom bedeutet und Y und Z die zur Formel I gegebenen Bedeutungen haben, wobei Thiolactimether der Formel XX erhalten werden

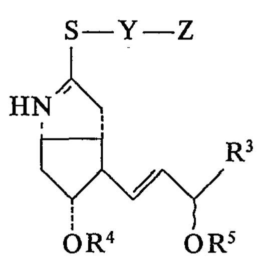

(XX)

worin Y, Z und $R^3$ die zur Formel I und $R^4$, $R^5$ die zur Formel XVII gegebenen Bedeutungen haben,

p) die Schutzgruppen an den Alkoholfunktionen in den Verbindungen der Formel XX durch basisch katalysierte Hydrolyse abspaltet, wodurch man zu Verbindungen der Formel I gelangt, worin X Schwefel bedeutet, oder

q) die Lactame der Formel XVII mit Alkylhalogeniden der Formel IXX alkyliert, wobei Lactimether der Formel XXI erhalten werden

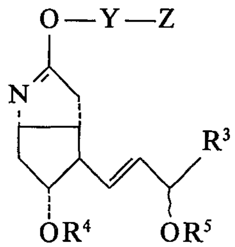

(XXI)

worin Y, Z und $R^3$ die zur Formel I gegebenen Bedeutungen haben, und $R^4$, $R^5$ die zur Formel XVII gegebene Bedeutung haben,

r) die Schutzgruppen an den Alkoholfunktionen in den Verbindungen der Formel XXI durch basisch katalysierte Hydrolyse abspaltet, wodurch man zu Verbindungen der Formel I gelangt, worin X Sauerstoff bedeutet, oder

s) die Thiolactame der Formel XVIII in die Alkylthiolactimether der Formel XXII überführt

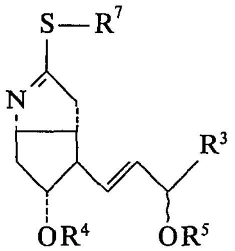

(XXII)

worin $R^3$ die zur Formel I und $R^4$, $R^5$ die zur Formel XVII gegebenen Bedeutungen haben und $R^7$ eine Methyl- oder Ethylgruppe bedeutet,

t) die Thiolactimether der Formel XXII mit Aminen der Formel XXIII umsetzt

$$Z{-}Y{-}NH_2 \qquad (XXIII)$$

worin Z und Y die zur Formel I gegebenen Bedeutungen haben zu Amidinen der Formel XXIV

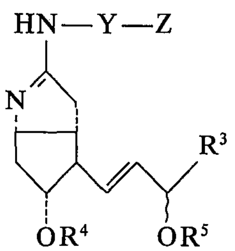

(XXIV)

worin Y, Z und $R^3$ die zur Formel I und $R^4$, $R^5$ die zur Formel XVII gegebenen Bedeutungen haben,

u) die Schutzgruppen an den Alkoholfunktionen in den Verbindungen der Formel XXIV durch basisch katalysierte Hydrolyse abspaltet, wodurch man zu Verbindungen der Formel I gelangt, worin X = NH ist,

v) gegebenenfalls Verbindungen der Formel XX, XXI und XXIV, worin $R^4$ und $R^5$ Wasserstoff bedeuten und worin Z Alkoxycarbonyl bedeutet, zu Verbindungen der Formel I verseift, worin $R^3$, X und Y die zur Formel I gegebenen Bedeutungen haben und Z Carboxyl oder ein Kation bedeutet,

w) gegebenenfalls eine Verbindung der Formel I, worin Z Carboxyl oder ein Kation bedeutet und $R^2$, X und Y die zur Formel I gegebenen Bedeutungen haben, zu einer Verbindung der Formel I verestert, worin $R^1$ einen Alkylrest mit der zur Formel I gegebenen Bedeutung ist und $R^3$, X und Y die zur Formel I gegebenen Bedeutungen haben,

y) gegebenenfalls in einer Verbindung der Formel I, worin Z Carboxyl oder ein physiologisch verträgliches Metall-, $NH_4$- oder Ammonium bedeutet, das sich von einem primären, sekundären oder tertiären Amin ableitet, und $R^1$, X und Y die zur Formel I gegebenen Bedeutungen haben, das Kation $R^1$ gegen ein anderes austauscht.

Für die Herstellung des im erfindungsgemäßen Verfahren als Ausgangsmaterial verwendeten Alkohols II kann man analog dem Verfahren arbeiten, das in J. Am. Chem. Soc. 95, 7522 (1973) beschrieben ist.

Die Herstellung des Benzylethers erfolgt durch übliche Methoden, indem man den Alkohol der Formel II in inerten Lösungsmitteln, wie z. B. Dimethylformamid, Dimethoxyethan oder Toluol mit Benzylbromid in Gegenwart von Basen wie z. B. Natriumhydrid, Bariumhydroxid oder Kaliumcarbonat bei

Temperaturen von 20—140°C zur Reaktion bringt. Man kann aber auch auf das inerte Lösungsmittel verzichten und die Reaktion in Benzylbromid ablaufen lassen.

Das Lacton der Formel III läßt sich mit Ammoniak, z. B. in alkoholischer Lösung bei erhöhter Temperatur (70—130°C) zum Hydroxyamid der Formel IV umsetzen. Diese Reaktion wird zweckmäßigerweise im Autoklaven durchgeführt. An dieser Stelle ist es vorteilhaft, das Produkt durch Chromatographie an Kieselgel zu reinigen.

Die Oxidation des so erhaltenen Hydroxyamids der Formel IV zum Ketoamid der Formel V kann mit Oxidationsmitteln wie Chromtrioxid/Dimethylsulfat, Chromtrioxid/Pyridin, Pyridiniumchlorochromat, Pyridiniumdichromat, Chromtrioxid/Schwefelsäure/Wasser in inerten Lösungsmitteln wie Dimethylformamid, Methylenchlorid oder Aceton bei Temperaturen von —30° bis 40°C, bevorzugt zwischen —25 und —20°C, durchgeführt werden. Das Ketoamid der Formel V kann an dieser Stelle durch Chromatographie an Kieselgel gereinigt werden.

Das Ketoamid der Formel V steht im tautomeren Gleichgewicht mit seinem cyclischen Isomeren, dem Hydroxylactam der Formel VI. Solche Gleichgewichte sind aus der Literatur bekannt, z. B. Chem. Ber. 103, 3205 (1970).

Löst man das Ketoamid der Formel V in einem inerten Lösungsmittel, z. B. Aceton, Methanol, Ethanol, Tetrahydrofuran, Chloroform oder Methylenchlorid und läßt die Lösung bei 20°—40°C stehen, erhält man das Hydroxylactam der Formel VI.

Das Hydroxylactam der Formel VI, das Ketoamid der Formel V oder Mischungen aus beiden lassen sich mit Mercaptanen der Formel VII und Chlortrimethylsilan in inerten Lösungsmitteln, wie z. B. Methylenchlorid, Chloroform, Toluol oder Dimethoxyethan zu Thioethern der Formel VIII umsetzen. Die Reaktion wird bei 30° bis 100°C durchgeführt, zweckmäßigerweise in Gegenwart einer organischen Base, wie z. B. Pyridin, Triethylamin oder 1,4-Diazabicyclo [4,3,0]nonen-5 (DBN).

Die Reduktion der Thioether der Formel VIII erfolgt durch Umsetzung mit einem Metallkatalysator wie z. B. Pd/C, Raney-Ni oder $NiCl_2/NaBH_4$ und Wasserstoff in einem niedrig-Alkanol, wie z. B. Methanol, Ethanol, t-Butanol oder iso-Propanol oder in Aceton.

Durch Erwärmen erhält man den Benzylether der Formel IX.

Der Benzylether der Formel IX läßt sich nun mit denselben Katalysatoren wie bei der Umsetzung der Thioether der Formel VIII beschrieben mit Wasserstoff in einem niedrig-Alkanol, z. B. Methanol, i-Propanol oder Butanol, THF oder Essigester zum Alkohol der Formel X umsetzen. Dabei können dem inerten Lösungsmittel 5—10% einer Mineralsäure, z. B. konz. Chlorwasserstoffsäure, oder einer org. Säure wie z. B. Essigsäure zugesetzt werden. Man kann dabei bei Normaldruck und Raumtemperatur arbeiten oder die Reaktion im Autoklaven bei 20—80°C unter einem Druck von 50—100 atm. durchführen.

Man kann aber auch die Thioether der Formel VIII mit einem großen Überschuß der oben beschriebenen Katalysatoren bei 20—80°C mit Wasserstoff behandeln, wobei gleichzeitig die $R^3$—S und die Benzylethergruppierung abgespalten werden und man so direkt den Alkohol der Formel X erhält.

Die Herstellung der Verbindungen der Formel XI erfolgt durch Umsetzung des Alkohols der Formel X entweder mit Anhydriden der Formel $R^4$—O—$R^4$ oder Säurechloriden der Formel $R^4$—Cl, wobei $R^4$ eine Acylgruppe bedeutet. Unter den Acylgruppen sind die im folgenden aufgeführten Substituenten besonders bevorzugt:

Acetyl, Propionyl, Benzoyl, subst. Benzoyl (z. B. 3-Methylbenzoyl, 4-Phenylbenzoyl, 2,4-Dinitrobenzoyl, 2-Nitrobenzoyl, 1-Napththoyl, 2-Naphthoyl). Die Reaktion wird im Falle der Anhydride ohne Lösungsmittel oder im Falle der Umsetzung mit Säurechloriden in inerten Lösungsmitteln wie z. B. Chloroform, Methylenchlorid, Tetrachlorkohlenstoff, Tetrahydrofuran oder Dioxan in Gegenwart einer Base durchgeführt. Als Basen eignen sich z. B. Pyridin, Triethylamin oder 4-Dimethylaminopyridin.

Die Tetrahydropyranosylgruppe in Verbindungen der Formel XI läßt sich durch sauer katalysierte, selektive Hydrolyse abspalten. Als Säuren eignen sich verdünnte Mineralsäuren oder organische Säuren, wie z. B. p-Toluolsulfonsäure, Oxalsäure oder Essigsäure, die in inerten Lösungsmitteln wie Methanol, Ethanol, Chloroform, Methylenchlorid, Tetrahydrofuran oder Toluol bei 0—50°C zur Reaktion gebracht werden.

Die Oxidation der Alkohole der Formel XII zu Aldehyden der Formel XIII kann durch Oxidationsmittel wie Pyridiniumchlorochromat, Pyridiniumdichromat in inerten Lösungsmitteln wie Methylenchlorid oder Chloroform erfolgen. Eine weitere Möglichkeit der Oxidation besteht in der Reaktion mit Thioanisid/$Cl_2$/Trimethylamin in Tetrachlorkohlenstoff. Der Aldehyd der Formel XIII wird zweckmäßigerweise ohne weitere Reinigung weiterverarbeitet.

Im weiteren werden die Aldehyde der Formel XIII nach Horner-Emmons-Wittig mit den Phosphonsäureestern der Formel XIV zu den ungesättigten Ketonen der allg. Formel XV umgesetzt, wobei eine bevorzugte Ausführungsform darin besteht, daß man das Natriumsalz des Phosphonsäureesters der Formel XIV mit Natriumhydrid in Dimethoxyethan herstellt und anschließend den Aldehyd der Formel XIII zugibt und bei Raumtemperatur 2—6 Stunden reagieren läßt. Die Phosphonsäureester der Formel XIV werden nach literaturbekannten Verfahren (siehe z. B. J. Am. Chem. Soc. 88, 5654 (1966) hergestellt.

Die Alkohole der Formel XVI erhält man in Form ihrer Epimerengemische, wenn man die Ketone der Formel XV mit einem komplexen Metallhydrid, vorzugsweise einem Alkaliboranat oder mit D,L-Isobor-

nyloxyaluminium-isopropoxid reduziert.

Die Alkoholfunktion in Verbindungen der Formel XVI läßt sich analog dem Verfahren, das bei der Herstellung von Verbindungen der Formel XI beschrieben wurde, mit den dort beschriebenen Schutzgruppen schützen. Man kann aber auch Silylschutzgruppen verwenden. Dazu wird der Alkohol der Formel XVI in einem inerten Lösungsmittel, wie z. B. Chloroform, Methylenchlorid oder Toluol bei 0–30°C mit dem Silylchlorid, bevorzugt Dimethyl-t-butylsilylchlorid, und einer Base zur Reaktion gebracht. Als Basen eignen sich z. B. Triethylamin, Pyridin oder 1,5-Diazabicyclo[5,4,0]undecen-5 (DBU).

Die Thiolactame der Formel XVIII lassen sich aus den Lactamen der Formel XVII durch Umsetzung mit schwefelübertragenden Reagentien, wie z. B. Phosphorpentasulfid, Phosphorpentasulfid/Calciumoxid, Phosphorpentasulfid-Pyridin-Komplex oder Phosphorpentasulfid-Anisol-Komplex in inerten Lösungsmitteln, wie z. B. Toluol, Dimethoxyethan oder Pyridin nach literaturbekannten Methoden herstellen (siehe z. B. Bull. Soc. Chim. Belg. 87, [3], 229 [1978]).

Zur Darstellung von Thiolactimethern der Formel XX werden die Thiolactame der Formel XVIII mit Alkylhalogeniden der Formel IXX alkyliert. Diese Reaktion wird in einem inerten Lösungsmittel, wie z. B. Toluol, Tetrahydrofuran, Dimethoxyethan oder Dimethylformamid in Gegenwart von Basen, wie z. B. Pyridin, Triethylamin, Kaliumcarbonat oder Natriumhydrid bei 15–55°C durchgeführt. Eine bevorzugte Ausführungsform dieser Reaktion besteht darin, daß man das Natriumsalz der Thiolactame der Formel XVIII mit Natriumhydrid in Dimethoxyethan herstellt, und dazu eine Lösung des Alkylhalogenids der Formel XIX in Dimethoxyethan tropft und 2–6 Stunden bei 25°C rührt.

Die Darstellung von Lactimethern der Formel XXI erfolgt durch Alkylierung der Lactame der Formel XVII mit Alkylhalogeniden der Formel XIX in einem inerten Lösungsmittel wie Benzol, Toluol, Dioxan oder Xylol bei Temperaturen von 80 bis 160°C in Gegenwart einer anorganischen Base, bevorzugt Silberoxid oder Silberhydroxid.

Zur Darstellung der Amidine der Formel XXIV wird das Thiolactam der Formel XVIII zu einer S-Alkylverbindung der Formel XXII alkyliert. Dabei kann man wie bei der Herstellung der Thiolactimether der Formel XV arbeiten, indem man zunächst mit Natriumhydrid in einem inerten Lösungsmittel wie Dimethoxyethan, Tetrahydrofuran oder Dioxan das Natriumsalz des Thiolactams herstellt und dann mit einem Alkylhalogenid der Formel $R^6$-Halogen, wobei $R^6$ Methyl oder Ethyl und Halogen Jod, Brom oder Chlor, bevorzugt aber Jod bedeutet, umsetzt. Man kann aber auch das Thiolactam der Formel XVIII mit dem Alkylhalogenid der Formel $R^2$-Halogen in einem inerten Lösungsmittel wie Aceton, Essigester oder Chloroform umsetzen, und den Thiolactimether der Formel XXII mit Basen wie Natriumhydrogencarbonat, Natrium- oder Kaliumcarbonat freisetzen.

Durch Erhitzen der Thiolactamether der Formel XXII mit Aminen der Formel XXIII in einem Niedrigalkanol, wie z. B. Methanol oder Ethanol oder in inerten Lösungsmitteln wie z. B. Dioxan oder Aceton erhält man die Amidine der Formel XXIV.

Die Schutzgruppen $R^4$ und $R^5$ in den Verbindungen der Formeln XX, XXI oder XXIV können unter milden alkalischen Bedingungen, z. B. mit Natrium- oder Kaliumcarbonat in alkoholischer Lösung abgespalten werden. Man arbeitet bei −10 bis +30°C und erhält Verbindungen der Formel I.

Verbindungen der Formel I, worin Z eine Alkoxycarbonylgruppe bedeutet, können in üblicher Weise in alkalischem Medium zu Verbindungen der Formel I, worin Z eine Carboxylgruppe bedeutet, verseift werden, z. B. mit NaOH oder KOH in einem niedermolekularen Alkohol wie Methanol oder Äther wie Dimethoxyäther oder THF, ggf. in Gegenwart von Wasser. Vorteilhafterweise benutzt man die äquimolare Menge oder einen sehr geringen Überschuß an Alkalihydroxyd, so daß man das Alkalisalz der Formel I ($R^1$ = Alkalimetallion) durch Verdampfen des Lösungsmittels erhält, vorzugsweise durch Gefriertrocknung.

Das Alkalikation läßt sich an Ionenaustauschern in üblicher Weise gegen beliebige Kationen austauschen. Dazu läßt man die Lösung des Alkalisalzes eines erfindungsgemäßen Hetero-Imino-Prostacyclinderivates durch eine mit einem Kationenaustauscher, wie z. B. Amberlite CG-50 oder Dowex CCR-2, gefüllte Säule laufen.

Der Kationenaustauscher ist mit dem erwünschten Kation beladen, z. B. mit einem Ammoniumion, das sich von einem primären, sekundären oder tertiären Amin ableitet. Das erwünschte Salz erhält man durch Eindampfen des Eluats.

Man kann Verbindungen der Formel I, bei denen Z eine Carboxylgruppe und $R^1 = NH_4$ oder ein Ammoniumion bedeutet, das sich von einem primären, sekundären oder tertiären Amin ableitet, auch herstellen, indem man Verbindungen der Formel I, bei denen Z Carboxyl und $R^1$ = Wasserstoff bedeutet, in einer alkoholischen Lösung mit einer äquimolaren Menge des entsprechenden Amins versetzt und das Lösungsmittel eindampft.

Verbindungen der Formel I, worin Z eine Carboxy- oder Carboxylatgruppe ($R^1$ Wasserstoff oder ein Kation) bedeutet und $R^2$ die zur Formel I angegebenen Bedeutungen hat, lassen sich zu Verbindungen der Formel I verestern, worin $R^1$ einen Alkylrest bedeutet. So kann man z. B. ein Hetero-Imino-Prostacyclinderivat der Formel I ($R^1$ = H) bei Temperaturen zwischen −40 und +20° mit Diazoalkanen der Formel $R^1 - N_2$ ($R^1$ = Alkyl) verestern, wobei die üblichen Lösungsmittel wie z. B. Diäthyläther, Tetrahydrofuran, Chloroform oder nieder-molekulare Alkohole wie Methanol verwendet werden können. Die resultierenden Ester können in einfacher Weise durch Eindampfen des Lösungsmittels isoliert und ggf. chromatographisch gereinigt werden. Eine bevorzugte Veresterungsmethode besteht darin, daß man das

Salz des entsprechenden Hetero-Imino-Prostacyclinderivates I ($R^1$ = Kation) in Gegenwart einer Base wie z. B. eines Metallalkoholates oder Metallcarbonates in einem geeigneten Lösungsmittel mit einem Alkylierungsmittel $R^1 - Z'$ umsetzt. Als Metallalkoholate kommen z. B. Natriummethylat, Natriumäthylat oder Kaliumtertiärbutylat in Betracht, als Carbonate eignen sich z. B. Calciumcarbonat oder Natriumhydrogencarbonat. Als geeignetes Lösungsmittel kommen Alkohole wie z. B. Methanol oder tert. Butanol, Äther wie Tetrahydrofuran oder 1,2-Dimethoxyäther und insbesondere dipolare aprotische Lösungsmittel wie Dimethylformamid, Dimethylsulfoxyd, Acetonitril, oder N'-Methylpyrrolidon in Betracht. In den Alkylierungsmitteln $R^1 - Z'$ bedeutet Z' vorzugsweise Brom oder Jod oder einen Sulfonsäurerest.

Die Verbindungen der Formel XVI können als Diastereomerengemisch bezüglich der Stellung der Hydroxylgruppen am Kohlenstoffatom 15 (Prostaglandinnomenklatur), als reine $\alpha$- oder $\beta$-Isomere oder in Form von optisch aktiven Antipoden für die nachfolgenden Reaktionen eingesetzt werden. Die Trennung von Stereoisomeren bzw. Antipoden kann aber auch nach jeder folgenden Reaktionsstufe erfolgen. Das bedeutet, daß alle beschriebenen Reaktionen mit Diastereomerengemischen, reinen Diastereomeren oder optisch aktiven Antipoden durchgeführt werden können. Die beanspruchten Verbindungen der Formel I umfassen daher Diastereomerengemische, reine Diastereomere, Epimerengemische und reine Epimeren.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels z. B. Säulen-, Dünnschicht- oder Hochdruckflüssigkeitschromatographie.

Außer den in den Beispielen beschriebenen Verbindungen lassen sich nach den erfindungsgemäßen Verfahren auch noch folgende Verbindungen herstellen:

2-Aza-3-(1-aza-4-carboxybutyl-1)-6-(3-(R,S)-hydroxy-3-cyclohexyl-1-propenyl)-7-hydroxybicyclo[3,3,0]octen-2
2-Aza-3-(1-aza-4-carboxybutyl-1)-6-(3-(R,S)-hydroxy-6-oxa-1-octenyl)-7-hydroxybicyclo[3,3,0]octen-2
2-Aza-3-(1-aza-4-carboxybutyl-1)-6-(3-(R,S)-hydroxy-4,4-dimethyl-1-octenyl)-7-hydroxybicyclo[3,3,0]octen-2
2-Aza-3-(1-aza-4-carboxybutyl-1)-6-(3-(R,S)-hydroxy-3-phenoxymethyl-1-propenyl)-7-hydroxybicyclo[3,3,0]octen-2
2-Aza-3-(1-aza-4-carboxybutyl-1)-6-(3-(R,S)-hydroxy-3-(2,2,3,3-tetrafluorcyclobutylethyl)-1-propenyl)-7-hydroxybicyclo[3,3,0]octen-2
2-Aza-3-(1-aza-4-carboxybutyl-1)-6-(3-(R,S)-hydroxy-3-(2-chlor-3-thienyloxymethyl) 1-propenyl)-7-hydroxybicyclo[3,3,0]octen-2
2-Aza-3-(4-carboxymethylanilino)-6-(3-(R,S)-hydroxy-3-cyclohexyl-1-propenyl)-7-hydroxybicyclo[3,3,0]-octen-2
2-Aza-3-(4-carboxymethylanilino)-6-(3-(R,S)-hydroxy-6-oxa-1-octenyl)-7-hydroxybicyclo-[3,3,0]octen-2
2-Aza-3-(4-carboxymethylanilino)-6-(3-(R,S)-hydroxy-4,4-dimethyl-1-octenyl)-7-hydroxybicyclo[3,3,0]octen-2
2-Aza-3-(4-carboxymethylanilino)-6-(3-(R,S)-hydroxy-3-phenoxymethyl-1-propenyl)-7-hydroxybicyclo[3,3,0]octen-2
2-Aza-3-(4-carboxymethylanilino)-6-(3-(R,S)-hydroxy-3-(2,2,3,3-tetrafluorcyclobutylethyl)-1-propenyl)-7-hydroxybicyclo[3,3,0]octen-2
2-Aza-3-(4-carboxymethylanilino)-6-(3-(R,S)-hydroxy-3-(2-chlor-3-thienyloxymethyl)-1-propenyl)-7-hydroxybicyclo[3,3,0]octen-2
2-Aza-3-(1-thia-4-carboxymethylbutyl-1)-6-(3-(R,S)-hydroxy-6-oxa-1-octenyl)-7-hydroxybicyclo[3,3,0]octen-2
2-Aza-3-(1-thia-4-carboxymethylbutyl-1)-6-(3-(R,S)-hydroxy-4,4-dimethyl-1-octenyl)-7-hydroxybicyclo[3,3,0]octen-2
2-Aza-3-(1-thia-4-carboxymethylbutyl-1)-6-(3-(R,S)-hydroxy-3-phenoxymethyl-1-propenyl)-7-hydroxybicyclo[3,3,0]octen-2
2-Aza-3-(1-thia-4-carboxymethylbuten-3-yl-1)-6-(3-(R,S)-hydroxy-4,4-dimethyl-1-octenyl)-7-hydroxybicyclo[3,3,0]octen-2
2-Aza-3-(1-thia-4-carboxymethylbuten-3-yl-1)-6-(3-(R,S)-hydroxy-3-phenoxymethyl-1-propenyl)-7-hydroxybicyclo[3,3,0]octen-2
2-Aza-3-(1-oxa-4-carboxymethylbutyl-1)-6-(3-(R,S)-hydroxy-4,4-dimethyl-1-octenyl)-7-hydroxybicyclo[3,3,0]octen-2
2-Aza-3-(1-oxa-4-carboxymethylbutyl-1)-6-(3-(R,S)-hydroxy-3-phenoxymethyl-1-propenyl)-7-hydroxybicyclo[3,3,0]octen-2

Es sind u. a. aus der US-A-4 097 489, DE-A-2 909 361, Tetrahedron Lett. N° 16, pp. 1371—74, Negwer, Org.-Chem. Arzneimittel, Berlin 1978, Nr. 6547, S. 1251, Progress in Drug Research, Basel 1973, S. 469 (Bd. 17), Tetrahedron Lett. N° 9 (1979), S. 805—808 oder J. Am. Soc. 99 (23), Nov. 1977,

S. 7736—38 eine Reihe von durch Hetero-atomen durchbrochenen Prostaglandin- bzw. $PGI_2$-Analoga bekannt, welche jedoch die erfindungsgemäßen Verbindungen in ihrem untenstehenden Wirkprofil nicht nahezulegen vermögen.

Die erfindungsgemäßen Produkte der Formel I sind physiologisch sehr wirksam.

So sind diese Verbindungen z. B. wirksam bei der Inhibierung der Blutplättchen-Aggregation, der Verminderung der Hafteigenschaften der Blutplättchen und der Beseitigung oder Verhütung von Thromben bei Säugetieren, einschließlich Menschen. Die Substanzen können daher zur Behandlung und Verhütung von Myocard-Infarkten, zur Behandlung und Verhütung postoperativer Thromben, zum Offenhalten von implantierten Gefäßen und zur Behandlung von Krankheitszuständen wie Atherosklerose, Blutgerinnung durch Lipämie und andere klinischen Zuständen, bei welchen die zu Grunde liegenden Ätiologie mit einem Lipidungleichtgewicht oder Hyperlipidämie verbunden sind, verwendet werden. Weitere Anwendung in vivo sind bei geriatrischen Patienten die Verhütung von zentralen ischämischen Ausfällen und die Langzeit-Prophylaxe nach Mycoard-Infarkten und Schlaganfällen. Die Verbindungen werden zu diesem Zweck systematisch verabreicht, z. B. intravenös, subkutan, intramuskulär oder in Form steriler Implantate zur verlängerten Wirkung. Zur raschen Wirkung wird die intravenöse Verabreichung bevorzugt. Man verwendet Dosen von etwa 0,15—1,50 µg/kg Körpergewicht pro Tag, insbesondere 0,5—100 µg/kg sowie Einheitsdosen von etwa 0,0005 µg—2 mg (pro Patient) wobei die genaue Menge von Alter, Gewicht und Zustand des Patienten oder Tieres, der Häufigkeit und Art der Verabreichung abhängt.

Beim Zusatz dieser Verbindungen zu Gesamtblut gelangt man zu Anwendungen in vitro, wie z. B. bei der Lagerung von Gesamtblut zur Verwendung in Herz/Lungen-Maschinen. Das diese Verbindungen enthaltende Blut kann auch durch Organe, z. B. Herz und Nieren, zirkulieren, die einem Spender entnommen wurden und zur Verpflanzung bereitstehen. Die erfindungsgemäßen Verbindungen sind auch brauchbar zur Herstellung von an Blutplättchen reichen Konzentraten zur Verwendung bei Thrombocytopenie, Chemotherapie und Bestrahlungstherapie. Bei Anwendungen in vitro verwendet man 0,01—1,0 µg/ml Gesamtblut.

Insbesondere sind Verbindungen der Formel I auch brauchbar als hypotensive Mittel zur Herabsetzung des Blutdrucks bei Säugetieren einschließlich Menschen. Zu diesem Zweck erfolgt die Verabreichung oral in Dosen von etwa 1,5 µg—1,5 mg/kg, bevorzugt 5 µg—1 mg/kg Körpergewicht pro Tag bzw. als Einheitsdosis von etwa 50 µg—20 mg (pro Patient) oder durch intravenöse Infusion in einer Menge von etwa 0,001 bis etwa 1 µg/kg Körpergewicht pro Minute, oder in einer oder mehreren Dosen von etwa 1,5 bis 150 µg/kg Körpergewicht pro Tag.

Die Prostaglandinderivate sind auch brauchbar bei Säugetieren einschließlich Menschen sowie bestimmten Nutztieren, z. B. Hunden und Schweinen, zur Verminderung und Steuerung übermäßiger Magensaftsekretion, womit die Bildung von Magen/Darmgeschwüren vermindert oder vermieden werden und die Heilung solcher bereits vorhandener Geschwüre beschleunigt werden kann. Zu diesem Zweck werden die Verbindungen intravenös, subkutan oder intramuskulär injiziert oder infundiert. Das Dosierungsschema für das Prostaglandin hängt bei dieser Behandlung von verschiedenen Faktoren ab einschließlich Typ, Alter, Gewicht, Geschlecht und medizinischem Zustand des Patienten, dem Dosierungsschema des entzündungshemmenden Synthetase-Inhibitors und der Empfindlichkeit des Patienten auf den Synthetase-Inhibitor bezüglich der Magen/Darmwirkung. So empfindet z. B. nicht jeder Patient, der eine entzündungshemmende Substanz benötigt, die gleichen unangenehmen gastrointestinalen Effekte. Diese ändern sich vielmehr häufig in Art und Ausmaß. Es liegt daher im Erfahrungsbereich des Arztes oder Tierarztes, festzustellen, ob die Verabreichung der entzündungshemmenden Substanz unerwünschte gastrointestinale Effekte beim Mensch oder Tier erzeugt und die wirksame Menge des Prostaglandins zu verschreiben, mit der diese Effekte im wesentlichen eliminiert werden können.

Einzelne Vertreter dieser Substanzen eignen sich zur Behandlung von Asthma. Sie sind beispielsweise nützlich als Bronchiendilatoren oder als Inhibitoren von Mediatoren wie z. B. SRS-A und Histamin, die aus durch einen Antigen/Antikörper-Komplex aktivierten Zellen freigesetzt werden. Die Verbindungen bekämpfen daher Krämpfe und erleichtern das Atmen bei Krankheitszuständen wie Bronchialasthma, Bronchitis, Bronchiectase, Pneumonie und Emphysem. Für diese Zwecke werden die Verbindungen in verschiedenen Dosierungsformen verabreicht, z. B. oral in Form von Tabletten, Kapseln oder Flüssigkeiten, rektal in Form von Suppositorien, parenteral, subkutan oder intramuskulär, wobei intravenöse Verabreichung in Notsituationen bevorzugt wird.

Die wirksame Verabreichung beim Menschen erfolgt durch orale Inhalierung oder durch Aerosol-Inhalierung. Dosen von 0,01—10 µg/kg Körpergewicht, bevorzugt 0,05—2 µg/kg werden ein bis viermal täglich angewendet, wobei die genaue Menge von Alter, Gewicht und Zustand des Patienten und der Häufigkeit und Art der Verabreichung abhängt.

## Beispiel 1

### 2-Oxa-3-oxo-6-tetrahydropyranosyloxymethyl-7-benzyloxybicyclo[3.3.0]octan

Zu einer Lösung von 89.44 g Alkohol (0.35 mol) in 125 ml frisch destilliertem Benzylbromid (179.5 g,

1.05 mol) werden unter Argon und Kühlen 19.8 g NaH 55% (0.454 mol) portionsweise eingetragen. Nach Entfernen des Kühlbades heizt sich der Ansatz auf 105°C und wird dickflüssig. Unter Rühren wird noch eine Stunde auf 120°C geheizt, wobei die Lösung wieder dünnflüssig wird. Nach Abkühlen wird mit 200 ml Essigester verdünnt, und über Celite filtriert. (Der Rückstand wird vorsichtig in Ethanol eingerührt.) Das Filtrat wird eingeengt und der Rückstand im Vakuum destilliert. Zur Entfernung der letzten Reste Benzylbromid wird der Rückstand in 400 ml Essigester aufgenommen und mit 70 ml Triethylamin versetzt. Die nach Kühlung ausgefallenen Salze werden filtriert und das Filtrat eingeengt. Der Rückstand wird an der 3fachen Menge Kieselgel filtriert (Laufmittel: Cyclohexan/Essigester 1 : 1).

Ausbeute:
105 g (75%)
NMR ($CDCl_3$):
$\delta$ ppm: 7.3, s, 5H (Aryl-H), 4.8—5.2, m, 1H (O=C—O—C—H), 4.4—4.6, d (breit), 3H (Aryl-$CH_2$—O, O—CH—O)
IR (Film):
$cm^{-1}$: 3030—3050 (CH Aromat), 1760 (C = O Lacton)
Rf (Essigester):
0.43

## Beispiel 2

(1-Benzyloxy-2-tetrahydropyranosyloxymethyl-4-hydroxycyclopentyl-3)essigsäureamid

68.1 g Lacton (0.196 mol) Beispiel 1 werden in 250 ml Methanol und 200 ml flüssigem Ammoniak 16 Stunden bei 100°C im Schüttelautoklaven behandelt. Das Lösungsmittel wird abgezogen und der Rückstand an der 4fachen Menge Kieselgel filtriert (Laufmittel: Essigester, dann Essigester/Methanol).

Ausbeute:
49.3 g (69%)
Schmp.:
70—76°C (Essigester/Cyclohexan)
NMR ($CDCl_3$):
$\delta$ ppm: 7.3, s, 5H (Aryl-H), 5.4—6.3, m (breit), 2H ($NH_2$), 4.55, s, 2H (Aryl-$CH_2$—O), 4.1—4.3, m, 1H (O—CH—O)
IR (KBr):
$cm^{-1}$: 3050—3600 (breit, Schulter bei 3200 OH, NH), 3000—3050 (Aryl-H), 1670 (C=O Amid)
Rf (Essigester):
0.07

## Beispiel 3

(3-Tetrahydropyranosyloxymethyl-4-benzyloxycyclopentanon-1-yl-2)essigsäureamid

Zu einer Lösung von 49.3 g Alkohol (0.136 mol) (Beispiel 2) in 350 ml Aceton werden bei —20 bis —25°C 70 ml JONES-Lösung (= 19.32 g $CrO_3$, 0.193 mol) getropft. (26.7 g $CrO_3$/23 ml $H_2SO_4$ konz./21 ml $H_2O$ werden auf 100 ml aufgefüllt). Die Lösung wird noch 2 Stunden bei —20°C gerührt, und dann bei dieser Temperatur mit einem Überschuß an i-Propanol versetzt. Die Lösung wird mit Triethylamin neutralisiert, auf Raumtemperatur kommen lassen und über Celite filtriert. Der Rückstand wird gründlich mit Aceton gewaschen, das Filtrat weitgehend eingeengt und mit Essigester versetzt. Die organische Phase wird mehrmals mit halbges. NaCl-Lösung gewaschen, die vereinigten wäßrigen Phasen mit NaCl gesättigt und mehrmals mit Essigester extrahiert. Die vereinigten Extrakte werden über $MgSO_4$ getrocknet, eingeengt und über eine kurze Säule (5fache Menge Kieselgel, Laufmittel: Essigester) filtriert.

Ausbeute:
30 g (61.2%)
NMR ($CDCl_3$):
$\delta$ ppm: 7.3, s, 5H (Aryl-H), 5.2—6.1, m, 2H ($NH_2$), 4.55, m, 3H (Aryl-$CH_2$—O, O—CH—O), 3.25—4.2, m, 5H ($CH_2$—O)
IR (Film):
$cm^{-1}$: 3100—3600 (breit, Schulter bei 3200, OH, NH), 3000—3100 (Aryl-H), 1750 (C=O Fünfring), 1675 (C=O Amid)
Rf (Essigester/Methanol 1 : 1):
0.44

## Beispiel 4

### 1-Hydroxy-2-aza-3-oxo-6-tetrahydropyranosyloxymethyl-7-benzyloxybicyclo[3.3.0]octan

5,0 g Ketoamid (Beispiel 3) werden in 10 ml Methanol gelöst und bei Raumtemperatur stehengelassen. Der Reaktionsverlauf wird dünnschichtchromatographisch verfolgt und das Lösungsmittel nach Beendigung der Reaktion abgezogen.

Ausbeute:
 5,0 g (100%)
NMR ($CDCl_3$):
 $\delta$ ppm: 7.3, s, 5H (Aryl-H), 6.2–6.35, m, 1H (NH), 4.56, m, 3H (Aryl-$CH_2$–O, O–CH–O), 3.25–4.2, m, 5H ($CH_2$–O)
IR (Film):
 $cm^{-1}$: 3050–3600 (breit, Schulter bei 3200, NH, OH), 1680 (C=O Lactam)
Rf (Essigester/Methanol 8 : 1):
 0.35

## Beispiel 5

### 1-Phenylthio-2-aza-3-oxo-6-tetrahydropyranosyloxymethyl-7-benzyloxybicyclo[3.3.0]octan

29 g Ketoamid (80.3 mmol) (Beispiel 3) und 9.35 g Thiophenol (85 mmol) werden in 200 ml Pyridin/Methylenchlorid (1 : 1 vv) unter Argon gelöst. Zu dieser Lösung werden bei Raumtemperatur 14.05 g Trimethylchlorsilan (129 mmol, 16.4 ml) getropft. Danach wird die Lösung noch 6 Stunden bei einer Badtemperatur von 70°C unter Rückfluß gekocht. Nach Abkühlen wird das ausgefallene Pyridiniumhydrochlorid abgesaugt und das Filtrat eingeengt. Der Rückstand wird mit Essigester aufgenommen und mit Wasser gewaschen. Die wäßrige Phase wird noch 3mal mit Essigester extrahiert und die vereinigten organischen Phasen über $MgSO_4$ getrocknet. Nach Abziehen des Lösungsmittels verbleibt ein dunkles Öl, das ohne weitere Reinigung weiterverarbeitet werden kann.

Ausbeute:
 42.9 g (>100%)
NMR ($CDCl_3$):
 $\delta$ ppm: 7.2–7.5, m, 10H (Aryl-H), 6.15–6.4, m, 1H (NH), 4.35–4.6, m, 3H (Aryl-$CH_2$–O, O–CH–O), 3.2–4.2, m, 5H ($CH_2$–O)
IR (Film):
 $cm^{-1}$: 3200 (breit, NH) 3060 (Aryl-H), 1680 (C=O Lactam)
Rf (Essigester/Methanol 8 : 1):
 0,58

Nach demselben Verfahren lassen sich auch das Hydroxylactam (Beispiel 4) oder Mischungen aus Hydroxylactam und Ketoamid zum Thioether umsetzen.

## Beispiel 6

### 2-Aza-3-oxo-6-tetrahydropyranosyloxymethyl-7-benzyloxybicyclo[3.3.0]octan

42.9 g roher Thioether werden in 750 ml t-Butanol mit 200 g Raney-Nickel 30 min bei 25°C behandelt. Danach wird die überstehende Lösung über Celite filtriert und das Raney-Nickel mehrfach mit Methanol ausgekocht. Das Filtrat wird eingeengt und der Rückstand über eine kurze Säule mit Kieselgel (0.2–0.5 mm) filtriert (Laufmittel: Essigester/Methanol 2 : 1).

Ausbeute:
 20 g (95,2%)
NMR ($CDCl_3$):
 $\delta$ ppm: 7.2, s, 5H (Aryl-H), 6.85–7.2, m, 1H (NH) 4.35–4.55, m, 3H (Aryl-$CH_2$–O, O–CH–O), 3.1–4.2, m, 6H ($CH_2$–O, O=C–N–C–H)
IR (Film):
 $cm^{-1}$: 3200 (breit, NH), 1680 (C=O Lactam)
Rf (Essigester/Methanol 8 : 1):
 0.27

## Beispiel 7

### 2-Aza-3-oxo-6-tetrahydropyranosyloxymethyl-7-hydroxybicyclo[3.3.0]octan

10,1 g Benzylether (Beispiel 6) (29,24 mmol) werden mit 6 g Palladium/Kohle in 100 ml Essigester 24 Stunden bei Raumtemperatur und Normaldruck hydriert. Der Katalysator wird über Celite filtriert, das Filtrat eingeengt und der Rückstand an Kieselgel chromatographiert.

Ausbeute:
6.1 g (79.2%)
Schmp.:
105–108°C
NMR ($CDCl_3$):
$\delta$ ppm: 6.7–7.0, m, 1H (NH), 4.4–4.6, m, 1H (O–CH–O), 3.2–4.2, m, 7H ($CH_2$–O, OH, O=C–N–C–H)
IR (KBr):
$cm^{-1}$: 3500–3000 (breit, Schulter bei 3200, NH, OH), 1680 (C=O Lactam)
Rf (Essigester/Methanol 8 : 1):
0.10

## Beispiel 8

### 2-Aza-3-oxo-6-tetrahydropyranosyloxymethyl-7-biphenylcarbonyloxybicyclo[3.3.0]octan

6 g Alkohol (Beispiel 7) (17.4 mmol) werden mit 4.33 g p-Phenyl-benzoesäurechlorid (20 mmol) in 50 ml trockenem Pyridin bei Raumtemperatur gerührt. Das Pyridin wird abgezogen, der Rückstand wird in Essigester aufgenommen und die Lösung mit Wasser gewaschen. Die org. Phase wird über $MgSO_4$ getrocknet, eingeengt und aus Isopropanol kristallisiert.

Ausbeute:
9.1 g (89%)
NMR ($CDCl_3$):
$\delta$ ppm: 7.2–8.1, m, 9H (Aryl-H), 6.8–7.1, m, 1H (NH), 5.0–5.4, m, 1H (O=C–O–C–H), 4.35–4.55, m, 1H (O–CH–O), 3.2–4.2, m, 5H ($CH_2$–O, O=C–N–C–H)
Schmp.:
172–175°C
IR (KBr):
$cm^{-1}$: 3200 (breit, NH), 1720 (C=O Arylester), 1680 (C=O Lactam)
Rf (Essigester/Methanol 4 : 1):
0.45

## Beispiel 9

### 2-Aza-3-oxo-6-hydroxymethyl-7-biphenylcarbonyloxybicyclo[3.3.0]octan

9 g THP-Ether (20.7 mmol) werden mit 0.3 g p-Toluolsulfonsäure in 30 ml absolutem Methanol über Nacht bei Raumtemperatur gerührt. Die Lösung wird mit Pyridin neutralisiert und am Rotavapor eingeengt. Der Rückstand wird aus Methylenchlorid kristallisiert.

Ausbeute:
6.35 g (87.4%)
Schmp.:
70–72°C
NMR ($CDCl_3$):
$\delta$ ppm: 7.2–8.1, m, 9H (Aryl-H), 6.8–7.05, m, 1H (NH), 5.0–5.4, m, 1H (O=C–O–C–H), 3.95–4.3, m, 1H (O=C–N–C–H), 3.75, s, 1H (OH) 3.5, d, 2H ($CH_2$–O)
IR (KBr):
$cm^{-1}$: 3600–3000 (breit, Schulter bei 3200, NH, OH), 1720 (C=O Arylester), 1680 (C=O Lactam)
Rf (Essigester/Methanol 4 : 1):
0.32

## Beispiel 10

### 2-Aza-3-oxo-7-biphenylcarbonyloxybicyclo[3.3.0]octan-6-carbaldehyd

6 g Alkohol (Beispiel 9) (16.3 mmol) werden in 30 ml absolutem Methylenchlorid suspendiert und bei 0°C zu einer Suspension von 35,13 g Pyridiniumchlorochromat (163 mmol) und 100 g Celite in 150 ml absolutem Methylenchlorid getropft. Man läßt die Suspension auf Raumtemperatur kommen und rührt so lange bei Raumtemperatur, bis der Alkohol im DC (Laufmittel: Essigester) verschwunden ist. Danach wird die Suspension mit 16 g Natriumhydrogensulfat versetzt und noch 70 min bei Raumtemperatur gerührt. Danach wird über eine Fritte mit Magnesiumsulfat abgesaugt. Das Filtrat wird eingeengt und der Rückstand über eine kurze Säule mit Kieselgel filtriert.

Ausbeute:
4.05 g (67.8%)
NMR ($CDCl_3$):
$\delta$ ppm: 10.2, d, 1H (CHO), 7.3–8.3, m, 9H (Aryl-H), 6.5–6.7, m, 1H (NH), 4.9–5.2, m, 1H (O=C–O–CH) 3.7–4.0, m, 1H (O=C–N–C–H)
IR (KBr):
$cm^{-1}$: 3200 (breit, NH), 1740 (CHO), 1710 (C=O Arylester) 1660 (C=O Lactam)
Rf (Essigester/Methanol 8 : 1):
0.27

## Beispiel 11

### 2-Aza-3-oxo-6-(3-oxo-1-octenyl)-7-biphenylcarbonyloxybicyclo[3.3.0]octan

22 mg 55% NaH-Dispersion werden in 4 ml trockenem DME vorgelegt und 111 mg 2-Oxoheptylphosphorsäuredimethylester (0.5 mmol) zugetropft. Es bildet sich ein weißer Niederschlag. Zu dieser Suspension tropft man 150 mg Aldehyd (0.5 mmol) (Beispiel 10) in 3 ml DME. Dabei löst sich ein Teil des Niederschlags auf. Man läßt die Lösung über Nacht bei Raumtemperatur stehen. Das Lösungsmittel wird abgezogen und der Rückstand in Essigester aufgenommen. Nach Zugabe von Wasser wird mit Essigsäure auf pH 4–5 angesäuert, die organische Phase abgetrennt und die wäßrige Phase noch 3mal mit Essigester extrahiert. Die vereinigten Extrakte werden über $MgSO_4$ getrocknet und eingeengt. Es verbleibt ein fester Rückstand.

Ausbeute:
136 mg (72%)
Schmp.:
155°C (Essigester/Ether)
NMR ($CDCl_3$):
$\delta$ ppm: 7.2–8.1, m, 9H (Aryl-H), 6.0–7.0, m, 2H (H–C=C–H), 5.75, s (breit), 1H (NH), 5.1–5.4, m, 1H (O=C–O–C–H), 4.0–4.45, m, 1H (O=C–N–C–H)
IR (KBr):
$cm^{-1}$: 3200 (NH), 1715 (Arylester), 1690 (C=O Lactam, Enon), 1630 (C=C Enon), 1610 (C=C Aryl)
Rf (Essigester/Methanol 8 : 1):
0.49

In Analogie zu Beispiel 11 lassen sich durch Reaktion des Aldehyds (Beispiel 10) mit Phosphonaten der allgemeinen Formel XIV auch folgende Enone der allgemeinen Formel XV herstellen.

| Beispiel Nr. | $R^2$ = | NMR-Daten (δ ppm) |
|---|---|---|
| 11 a | 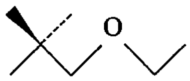 | 7.2–8.1, m, 9 H (Aryl—H),<br>6.05–7.0, m, 2 H (H—C=C—H),<br>5.8–5.9, m, 1 H (NH),<br>5.15–5.14, m, 1 H (O=C—O—C—H),<br>4.0–4.4, m, 1 H (O=C—N—C—H),<br>3.5, q, 2 H (O—$CH_2$—$CH_3$), 1.15,<br>t, 3 H ($CH_2$—$CH_3$) 0.9, s, 6 H (CH—$(CH_3)_2$) |
| 11 b | 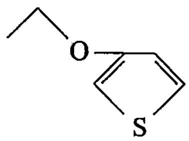 | 6.0–8.15, m, 15 H (Aryl—H, Thiophen—H,<br>H—C=C—H, NH),<br>5.1–5.4, m, 1 H (O=C—O—C—H),<br>4.0–4.4, m, 1 H (O=C—N—C—H),<br>3.9, d, 2 H ($CH_2$—O—Thiophen) |
| 11 c | 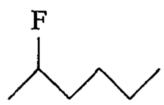 | 7.1–8.05, m, 9 H (Aryl—H),<br>6.05–7.0, m, 2 H (H—C=C—H),<br>5.9–6.0, m, 1 H (NH),<br>5.15–5.4, m, 1 H (O=C—O—C—H)<br>4.0–4.4, m, 1 H (O=C—N—C—H)<br>0.9, t, 3 H ($CH_2$—$CH_3$) |
| 11 d | 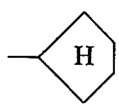 | 7.2–8.1, m, 9 H (Aryl—H),<br>6.05–7.05, m, 2 H (H—C=C—H),<br>6.0, s (breit), 1 H (NH),<br>5.2–5.4, m, 1 H (O=C—O—C—H),<br>4.0–4.4, m, 1 H (O=C—N—C—H) |
| 11 e | 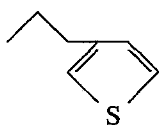 | 6.05–8.15, m, 14 H (Aryl—H—Thiophen—H,<br>H—C=C—H), 6.2, s (breit), 1 H (NH),<br>5.2–5.45, m, 1 H (O=C—OCH)<br>4.0–4.4, m, 1 H (O=C—N—C—H),<br>2.8, s (breit), 4 H (Thiophen—$CH_2$—$CH_2$) |
| 11 f | 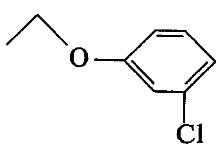 | 7.0–8.6, m, 13 H (Aryl—H),<br>6.1, s (breit), 1 H (NH),<br>5.2–5.4, m, 3 H (O=C—O—C—H,<br>Aryl—O—$CH_2$), 4.0–4.4, m, 1 H (O=C—N—C—H) |

Beispiel 12

2-Aza-3-oxo-6-(3-(R,S)-hydroxy-1-octenyl)-7-biphenylcarbonyloxybicyclo[3.3.0]octan

Zu einer Lösung von 22 mg $ZnBH_4$ (0.274 mmol) (hergestellt aus 37 mg $ZnCl_2$ und 10.4 mg $NaBH_4$ =je 0.274 mmol) in 5 ml trockenem DME werden bei 0°C 61 mg Enon (0.137 mmol) (Beispiel 11) in 2 ml DME getropft. Die Lösung wird noch 1 Stunde bei Raumtemperatur gerührt. Nach Einengen wird in Essigester aufgenommen und mit Wasser versetzt. Nach Ansäuern auf pH 3–4 wird die org. Phase abgetrennt, und die wäßrige Phase noch 2mal mit Essigester extrahiert. Die vereinigten org. Phasen werden über $MgSO_4$ getrocknet und eingeengt. Der verbleibende Feststoff wird aus Essigester kristallisiert.

Ausbeute:
50 mg (81.6%)
NMR ($CDCl_3$):
δ ppm: 7.3–8.1, m, 9H (Aryl-H), 5.5–5.7, m, 3H (H–C=C–H, NH), 5.0–5.4, m, 1H (O=C–O–C–H), 3.9–4.4, m, 2H (O=C–N–C–H, CH–OH)

IR (KBr):
cm$^{-1}$: 3400 (breit OH), 3200 (breit NH), 1720 (C=O Arylester), 1685 (C=O Lactam), 1615 (C=C Aromat)
Schmp.:
177°C (Essigester)
Rf (Essigester/Methanol 8 : 1):
0.35 ($\beta$-Isomeres), 0.28 ($\alpha$-Isomeres)

In Analogie zu Beispiel 12 lassen sich aus den Verbindungen der Beispiele 11a—11f durch Reduktion die Verbindungen 12a—12f (Formel XVI) herstellen.

| Beispiel 12 | $R^2$ | Rf-Werte ($\beta/\alpha$) (Essigester/Methanol 8 : 1) |
|---|---|---|
| a) | 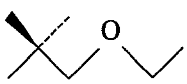 | 0.32/0.26 |
| b) | 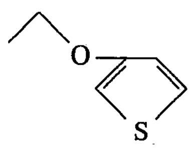 | 0.35/0.30 |
| c) | 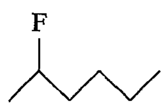 | 0.29/0.27 |
| d) | 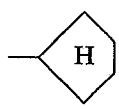 | 0.30/0.27 |
| e) | 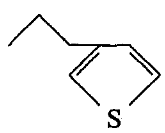 | 0.31/0.30 |
| f) | 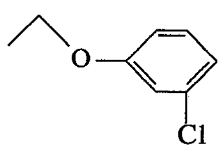 | 0.33/0.29 |

## Beispiel 13

### 2-Aza-3-oxo-6-(3-(R,S)biphenylcarbonyloxy-1-octenyl)-7-biphenylcarbonyloxybicyclo[3.3.0]octan

46 mg Alkohol (0.103 mmol) (Beispiel 12) und 24 mg p-Phenylbenzoylchlorid (0.110 mmol) werden in 1 ml trockenem Pyridin bei Raumtemperatur über Nacht gerührt. Das Pyridin wird abgezogen, und der Rückstand in Essigester aufgenommen. Nach Extraktion mit Wasser und Trocknen über $MgSO_4$ wird eingeengt. Der Rückstand wird an Kieselgel (Laufmittel: Essigester) chromatographiert.

Ausbeute:
56 mg (87%)
Schmp.:
132—133°C (Essigester/Hexan)
NMR ($CDCl_3$):
$\delta$ ppm: 7.2—8.1, m, 18H (Aryl-H), 5.95, s (breit), 1H (NH), 5.55—5.7, m, 2H (H—C=C—H), 4.9—5.55, m, 2H (O=C—O—C—H), 3.9—4.35, m, 1H (O=C—N—C—H)

IR (KBr):
cm$^{-1}$: 3200 (breit, NH) 1715 (C=O Arylester), 1690 (Schulter, C=O Lactam), 1615 (C=C Aromat)
Rf (Essigester):
0.29

In Analogie zu Beispiel 13 lassen sich aus den Verbindungen der Beispiele 12a—12f durch Veresterung die Diester XVII ($R^4$ und $R^5$ = Biphenylcarbonyl) herstellen.

| Beispiel 13 | $R^2$ | Rf-Werte (Essigester) |
|---|---|---|
| a) | 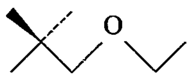 | 0.31 |
| b) | 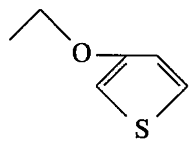 | 0.33 |
| c) | 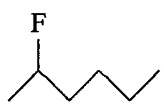 | 0.30 |
| d) | 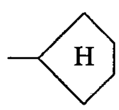 | 0.32 |
| e) | 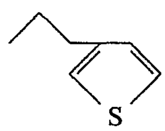 | 0.32 |
| f) | 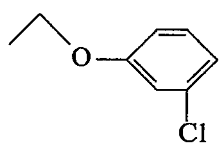 | 0.34 |

## Beispiel 14

### 2-Aza-3-thio-6-(3-(R,S)biphenylcarbonyloxy-1-octenyl)-7-biphenylcarbonyloxybicyclo[3.3.0]octan

36 mg Lactam (0,057 mmol) (Beispiel 13) werden mit 65 mg $P_2S_5$-Pyridin Komplex (0,085 mmol) in 1 ml trockenem Pyridin 3 Stunden auf 80°C erhitzt. Die Lösung wird eingeengt und der Rückstand in Essigester aufgenommen. Die Lösung wird 2mal mit 1/2 ges. NaCl-Lösung gewaschen, die wäßrige Phase mit NaCl gesättigt und noch 3 mal mit Essigester extrahiert. Die vereinigten org. Phasen werden über $MgSO_4$ getrocknet, eingeengt und das verbleibende Öl über eine Kieselgelsäule (Laufmittel: Essigester) filtriert.

Ausbeute:
30 mg (81.3%)
NMR ($CDCl_3$):
$\delta$ ppm: 7.15—8.2, m, 18H (Aryl-H), 5.55—5.8, m, 2H (H—C=C—H), 5.0—5,55, m, 2H (O=C—O—C—H), 4.2—4.6, m, 1H (O=C—N—C—H)
IR (Film):
cm$^{-1}$: 3300, 3150 (NH), 3030, 3060 (CH Aryl), 1715 (C=O Arylester), 1610 (C=C Aromat), 1510 (R—NH—C=S)
Rf (Cyclohexan/Essigester 1 : 1):
0.37 ($\beta$-Isomeres), 0.30 ($\alpha$-Isomeres)

In Analogie zu Beispiel 14 lassen sich aus den Verbindungen der Beispiele 13a—13f die Thiolactame XVIII ($R^4$ und $R^5$ = Biphenylcarbonyl) herstellen.

| Beispiel 14 | $R^2$ | Rf-Werte (β/α) (Essigester/Cyclohexan 1:1) |
|---|---|---|
| a) | 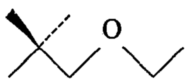 | 0.32/0.26 |
| b) | 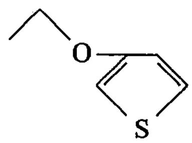 | 0.34/0.29 |
| c) | 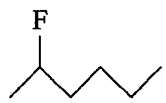 | 0.35/0.30 |
| d) | 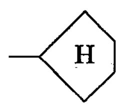 | 0.34/0.28 |
| e) | 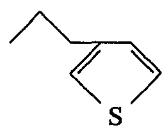 | 0.36/0.30 |
| f) | 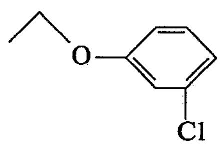 | 0.35/0.30 |

## Beispiel 15

2-Aza-3-(1-thia-4-carboxyethylbutyl-1)-6-(3-(R,S)biphenylcarbonyloxy-1-octenyl)-7-biphenylcarbonyloxybicyclo[3.3.0]octen-2

2 mg NaH 55%Dispersion (= 1,1 mg NaH, 0,046 mmol) werden in 0.5 ml trockenem DME vorgelegt. Bei Raumtemperatur werden 28 mg Thiolactam (0.044 mmol) (Beispiel 14) in 0.5 ml DME zugetropft und so lange gerührt, bis die Wasserstoffentwicklung beendet ist. Danach tropft man 9 mg Brombuttersäureester (0.046 mmol, 6.7 μl) zu, und rührt bei Raumtemperatur über Nacht. Das Lösungsmittel wird abgezogen und der Rückstand an Kieselgel (Laufmittel: Essigester/Cyclohexan 1 : 1) chromatographiert.

Ausbeute:
22.8 mg (69.2%)
NMR ($CDCl_3$):
δ ppm: 7.6—8.1, m, 18H (Aryl-H), 5.5—5.7, m, 2H (H—C=C—H), 4.75—5.5, 2H (O=C—O—C—H), 4.25—4.75, m, 1H (S—C=N—C—H), 4.05, q, 2H (J=7Hz, O=C—O—$CH_2$), 3.05, t, 2H (N=C—S—$CH_2$)
IR (Film):
$cm^{-1}$: 1730 (C=O Ester), 1600 (C=N)
Rf (Essigester/Cyclohexan 1 : 1):
0.46

In Analogie zu Beispiel 15 lassen sich aus den Verbindungen der Beispiele 14a—14f durch Alkylierung die Thiolactimether XX ($R^4$ und $R^5$ = Biphenylcarbonyl, Z = $CO_2C_2H_5$, Y = $-(CH_2)_3$ herstellen.

| Beispiel 15 | $R^2$ | Rf-Werte (Essigester/Cyclohexan 1:1) |
|---|---|---|
| a) | 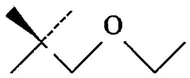 | 0.43 |
| b) | 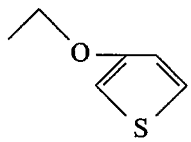 | 0.45 |
| c) | 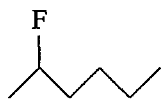 | 0.44 |
| d) | 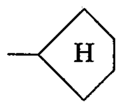 | 0.42 |
| e) | 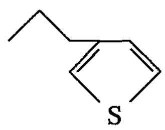 | 0.46 |
| f) | 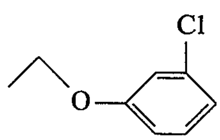 | 0.42 |

## Beispiel 16

2-Aza-3-(1-thia-4-carboxymethylbutyl-1)-6-(3-(R,S)-hydroxy-1-octenyl)-7-hydroxy-bicyclo[3.3.0]octen-2

10 mg PB-Ester (0.0132 mmol) (Beispiel 15) werden mit 11 mg Kaliumcarbonat (0.08 mmol) in 0.5 ml trockenem Methanol über Nacht bei Raumtemperatur gerührt. Die Lösung wird auf 0°C gekühlt und mit ges. Citronensäurelösung auf pH 4—5 gestellt. Der Niederschlag wird abgesaugt und das Filtrat nach Entsäuern mit ges. $NaHCO_3$-Lösung eingeengt. Der Rückstand wird in Essigester aufgenommen, mit NaCl-Lösung versetzt, die org. Phase abgetrennt und die wäßrige Phase mit Essigester extrahiert. Nach Trocknen und Einengen wird an Kieselgel (Laufmittel: Methylenchlorid/Methanol 10 : 1) chromatographiert.

Ausbeute:
4.4 mg (87.2%)

NMR ($CDCl_3$):
$\delta$ ppm: 5.4—5.6, m, 2H (H—C=C—H), 4.3—4.55, m, 1H (S—C=N—C—H), 3.6—4.3, m, 2H (CH—CH), 3.6, s, 3H ($COOCH_3$), 3.1, t, 2H (N=C—S—$CH_2$)

IR (Film):
$cm^{-1}$: 1720 (C=O Ester), 1600 (C=N)

Rf (Methylenchlorid/Methanol 10 : 1):
0.69 ($\beta$-Isomeres)
0.52 ($\alpha$-Isomeres)

In Analogie zu Beispiel 16 lassen sich aus den Verbindungen der Beispiele 15a—15f durch Verseifung die Thiolactimether der Formel I (X = S, Y = $-(CH_2)_3-$, Z = $CO_2CH_3$) herstellen.

| Beispiel 16 | $R^2$ | Rf-Werte (β/α) (Methylenchlorid/Methanol 10 : 1) |
|---|---|---|
| a) | 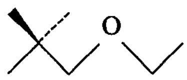 | 0.35/0.21 ($CH_2CH_2/CH_3OH$ 15 : 1) |
| b) | 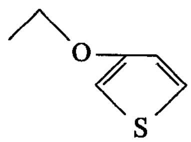 | 0.65/0.50 |
| c) | 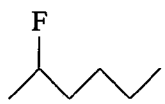 | 0.60/0.44 |
| d) | 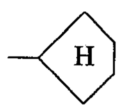 | 0.62/0.46 |
| e) | 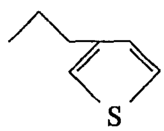 | 0.67/0.53 |
| f) | 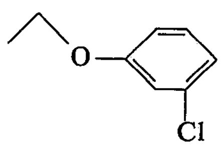 | 0.21/0.14 (Cyclohexan/Essigester 1 : 1) |

## Beispiel 17

2-Aza-3-(1-thia-4-carboxyethylbuten-3-yl-1)-6-(3-(R,S)-biphenylcarbonyloxy-1-octenyl)-7-biphenylcarbonyloxybicyclo[3.3.0]octen-2

2 mg NaH 55% Dispersion (= 1.1 mg NaH, 0.046 mmol) werden in 0.5 ml trockenem DME vorgelegt. Bei Raumtemperatur werden 28 mg Thiolactam (0.044 mmol) (Beispiel 14) in 0.5 ml DME zugetropft und so lange gerührt, bis die Wasserstoffentwicklung beendet ist. Danach tropft man 8.9 mg Bromcrotonsäureester (0.046 mmol) zu und rührt über Nacht bei Raumtemperatur. Das Lösungsmittel wird abgezogen und der Rückstand an Kieselgel (Laufmittel: Essigester/Cyclohexan 1 : 1) chromatographiert.

Ausbeute:
18.4 mg (56%)
NMR ($CDCl_3$):
δ ppm: 7.6–8.1, m, 18H (Aryl-H), 5.85–7.1, m, 2H (H–C=CH–$CO_2$–), 4.25–4.75, m, 1H (S–C=N–C–H), 3.7, s, 3H ($CO_2CH_3$), 3.35, m, 2H ($CH_2$–S–C=N–)
IR (Film):
$cm^{-1}$: 1730 (C=O Ester), 1630 (C=N)
Rf (Essigester/Cyclohexan 1 : 1):
0.56

In Analogie zu Beispiel 17 lassen sich aus den Verbindungen der Beispiele 14a–14f durch Alkylierung die Thiolactimether XX ($R^4$ und $R^5$ = Biphenylcarbonyl, Z = $CO_2C_2H_5$, Y = $CH_2$–CH=CH–) herstellen.

| Beispiel 17 | $R^2$ | Rf-Werte (Cyclohexan/Essigester 1:1) |
|---|---|---|
| a) | 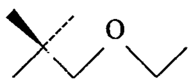 | 0.52 |
| b) | 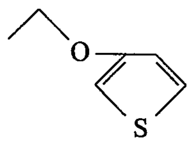 | 0.56 |
| c) | 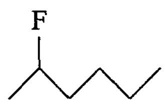 | 0.54 |
| d) | 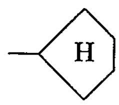 | 0.53 |
| e) | 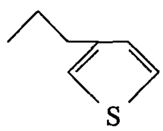 | 0.56 |
| f) | 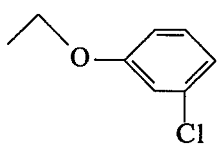 | 0.51 |

## Beispiel 18

2-Aza-3-(1-thia-4-carboxymethylbuten-3-yl-1)-6-(3-(R,S)-hydroxy-1-octenyl)-7-hydroxy-bicyclo[3.3.0]octen-2

20 mg PB-Ester (0.027 mmol) (Beispiel 17) werden mit 22 mg Kaliumcarbonat (0.16 mmol) in 1 ml trockenem Methanol über Nacht bei Raumtemperatur gerührt. Die Lösung wird auf 0°C gekühlt und mit ges. Citronensäurelösung auf pH 4—5 gestellt. Der Niederschlag wird abgesaugt und das Filtrat nach Entsäuern mit ges. $NaHCO_3$-Lsg. eingeengt. Der Rückstand wird mit Essigester aufgenommen, mit NaCl-Lsg. versetzt, die org. Phase abgetrennt und die wäßrige Phase noch mehrmals mit Essigester extrahiert. Nach Trocknen und Einengen wird an Kieselgel (Laufmittel: Methylenchlorid/Methanol 1 : 1) chromatographiert.

Ausbeute:
2.9 mg (56.3%)
NMR ($CDCl_3$):
$\delta$ ppm: 5.8—7.1, m, 2H (H—C=CH—$CO_2CH_3$), 5.5—5.7, m, 2H (H—C=C—H), 4.3—4.75, m, 1H (S—C=N—C—H), 3.6—4.3, m, 2H (CH—OH), 3.7, s, 3H ($CO_2CH_3$), 3.35, m, 2H ($CH_2$—S—C=N—)
IR (Film):
$cm^{-1}$: 3500—3000 (breit, OH), 1730 (C=O Ester), 1600 (C=N)
Rf (Methylenchlorid/Methanol 10 : 1):
0.70 ($\beta$-Isomeres)
0.61 ($\alpha$-Isomeres)

In Analogie zu Beispiel 18 lassen sich aus den Verbindungen der Beispiele 17a—17f durch Verseifung die Thiolactimether der Formel I (X=S, Y = —$CH_2$—CH=CH—, Z = $CO_2CH_3$) herstellen.

| Beispiel 18 | $R^2$ | Rf-Werte ($\beta/\alpha$) (Methylenchlorid/Methanol 10 : 1) |
|---|---|---|
| a) | 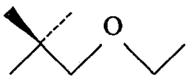 | 0.65/0.54 |
| b) | 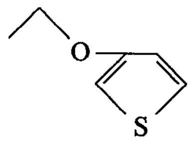 | 0.71/0.64 |
| c) | 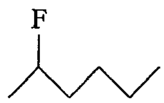 | 0.69/0.59 |
| d) | 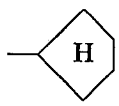 | 0.68/0.60 |
| e) | 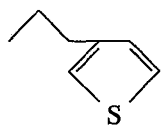 | 0.64/0.55 |
| f) | 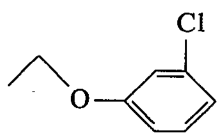 | 0.65/0.55 |

## Beispiel 19

2-Aza-3-(1-oxa-4-carboxyethylbutyl-1)-6-(3-(R,S)-biphenylcarbonyloxy-1-octenyl)-7-biphenylcarbonyloxybicyclo[3.3.0]octen-2

72 mg Lactam (0.114 mmol) (Beispiel 13) und 43,7 mg 4-Brombuttersäureethylester (0.228 mmol) werden in 2 ml trockenem Xylol gelöst. Die Lösung wird auf 60°C erwärmt, mit 39.4 mg Silberoxid (0.17 mmol) versetzt und 6 Stunden auf 140—160°C erwärmt. Nach Abkühlen wird mit 5 ml Diethylether versetzt, filtriert und das Filtrat eingeengt. Der Rückstand wird an Kieselgel (Laufmittel: Essigester/Cyclohexan 1 : 1) chromatographiert.

Ausbeute:
59,3 mg (69,9%)
NMR ($CDCl_3$):
$\delta$ ppm: 7.2—8.1, m, 18H (Aryl-H), 5.4—5.6, m, 2H (H—C=C—H), 4.6—5.4, m, 2H (O=C—O—C—H), 4.3, s (breit), 1H (O—C=N—C—H), 4.1 q (J = 3,5 Hz), 2H ($CO_2CH_2$—), 4.15, t (J = 3 Hz), 2H ($CH_2$— O—C=N—)
IR (Film):
$cm^{-1}$: 1735 (C=O Ester), 1645 (C=N)
Rf (Essigester/Methanol 8 : 1):
0.79

In Analogie zu Beispiel 19 lassen sich aus den Verbindungen der Beispiele 12a—12f die Lactimether XXI ($R^4$, $R^5$ = Biphenylcarbonyl, Z = $CO_2C_2H_5$, Y = $-(CH_2)_3-$) herstellen.

| Beispiel 19 | $R^2$ | Rf-Wert (Essigester/Methanol 8 : 1) |
|---|---|---|
| a) | 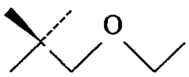 | 0.80 |
| b) | 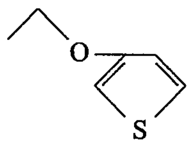 | 0.78 |
| c) | 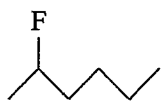 | 0.76 |
| d) | 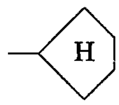 | 0.79 |
| e) | 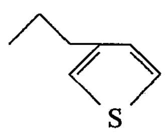 | 0.79 |
| f) | 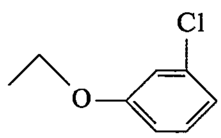 | 0.75 |

## Beispiel 20

2-Aza-3-(1-oxa-4-carboxymethylbutyl-1)-6-(3-(R,S)-hydroxy-1-octenyl)-7-hydroxybicyclo[3.3.0]octen-2

29.3 mg PB-Ester (0.0396 mmol) (Beispiel 19) werden mit 33 mg Kaliumcarbonat (0.24 mmol) in 1.5 ml trockenem Methanol über Nacht bei Raumtemperatur gerührt. Die Lösung wird auf 0°C gekühlt und mit ges. Citronensäurelösung auf pH 4—5 gestellt. Der Niederschlag wird abgesaugt, das Filtrat mit ges. $NaHCO_3$-Lsg. neutral gestellt und eingeengt. Der Rückstand wird in Essigester aufgenommen, mit ges. NaCl-Lsg. versetzt und die org. Phase abgetrennt. Die wäßrige Phase wird noch mehrmals mit Essigester extrahiert und die vereinigten Extrakte über $MgSO_4$ getrocknet und eingeengt. Der Rückstand wird an Kieselgel (Laufmittel: Essigester/Methanol 8 : 1) chromatographiert.

Ausbeute:
10.9 mg (67.2%)
NMR ($CDCl_3$):
$\delta$ ppm: 5.45—5.65, m, 2H (H—C=C—H), 3.9—4.45, m, 3H ($CH_2$—O—C=N, O—C=N—C—H), 3.6—3.9, m, 2H (CH—OH), 3.8, s, 3H ($CO_2CH_3$)
IR (Film):
$cm^{-1}$: 3600—3000 (breit, OH), 1730 (C=O Ester), 1640 (C=N)
Rf (Essigester/Methanol 8 : 1):
0.09 ($\beta$-Isomeres), 0.06 ($\alpha$-Isomeres)

In Analogie zu Beispiel 20 lassen sich auch die Verbindungen der Beispiele 19a—19f durch Verseifung in die Lactimether I (Z = $CO_2CH_3$, X = O, Y = $-(CH_2)_3-$) überführen.

| Beispiel 20 | $R^2$ | Rf-Wert ($\beta/\alpha$)<br>(Essigester/Methanol 8 : 1) |
|---|---|---|
| a) | 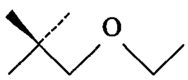 | 0.10/0.08 |
| b) | 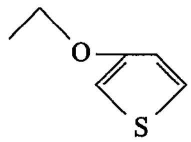 | 0.11/0.07 |
| c) | 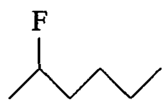 | 0.10/0.09 |
| d) | 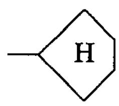 | 0.09/0.07 |
| e) | 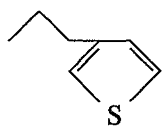 | 0.09/0.08 |
| f) | 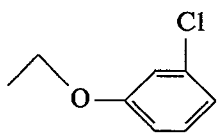 | 0.11/0.09 |

Beispiel 21

2-Aza-3-methylthio-6-(3-(R,S)-biphenylcarbonyloxy-1-octenyl)-7-biphenylcarbonyl-oxybicyclo[3.3.0]octen-2

129 mg Thiolactam (0.2 mmol) (Beispiel 14) werden in 1 ml trockenem DME gelöst und zu einer Suspension von 8.7 mg Natriumhydrid 55% Dispersion (0.2 mmol = 4.8 mg NaH) in 5 ml trockenem DME getropft. Es wird so lange bei Raumtemperatur gerührt, bis die Wasserstoffentwicklung beendet ist. Danach wird mit 31 mg Methyljodid (0.22 mmol) versetzt und die Lösung 4 Stunden auf 40°C erwärmt. Das Lösungsmittel wird abgezogen, der Rückstand in Diethylether aufgenommen, mit Wasser gewaschen, über $MgSO_4$ getrocknet und eingeengt.

Ausbeute:
95,5 mg (72.4%)
NMR ($CDCl_3$):
$\delta$ ppm: 7.6—8.1, m, 18H (Aryl-H), 5.5—5.7, m, 2H (H—C=—H), 4.75—5.5, m, 3H (O=C—O—C—H, S—C=N—C—H), 3.1, s, 3H ($CH_3$—S)
Rf (Essigester/Methanol 8 : 1):
0.83

Der Thiolactimether wird ohne weitere Reinigung für Folgereaktionen eingesetzt.
In Analogie zu Beispiel 21 lassen sich aus den Verbindungen der Beispiele 14a—14f durch Methylierung die Thiolactimether XXII ($R^7$ = $CH_3$, $R^4$, $R^5$ = Biphenylcarbonyl) herstellen.

| Beispiel 21 | $R^2$ | Rf-Werte (Essigester/Methanol 8 : 1) |
|---|---|---|
| a) | 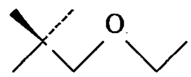 | 0.85 |
| b) | 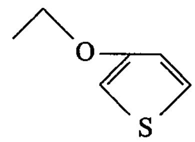 | 0.90 |
| c) | 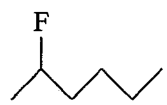 | 0.87 |
| d) | 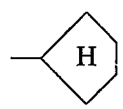 | 0.87 |
| e) | 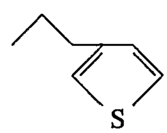 | 0.84 |
| f) | 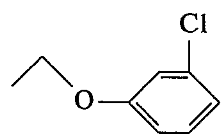 | 0.86 |

## Beispiel 22

**2-Aza-3-(1-aza-4-carboxybutyl-1)-6-(3-(R,S)-biphenylcarbonyloxy-1-octenyl)-7-biphenylcarbonyloxybicyclo[3.3.0]octen-2**

77 mg Thiolactimether (0.12 mmol) (Beispiel 21) werden mit 12.4 mg γ-Aminobuttersäure (0.12 mmol) in 1 ml Methanol 3 Stunden unter Rückfluß erhitzt. Das Lösungsmittel wird abgezogen und der Rückstand an Kieselgel (Laufmittel Methylenchlorid/Methanol 5 : 1) chromatographiert.

Ausbeute:
79.5 mg (95%)
NMR ($CDCl_3$):
δ ppm: 7.6–8.1, m, 18H (Aryl-H), 5.5–5.7, m, 3H (H–C=C–H, N=C–NH), 4.7–5.5, m, 2H (O=C–O–C–H), 4.35, m, 1H (N–C=N–C–H)
IR (Film):
$cm^{-1}$: 2500–3500 (Schulter bei 2980, $CO_2H$, CH, NH), 1680, 1565 (Amidin) 1565, 1400 (Carboxylat)
(Methylenchlorid/Methanol 1 : 2):
0.35

In Analogie zu Beispiel 22 lassen sich aus den Verbindungen der Beispiele 21a–21f die Amidine XXIV ($Z = CO_2H$, $R^4$, $R^5$ = Biphenylcarbonyl, $Y = -(CH_2)_3-$) herstellen.

| Beispiel 22 | $R^2$ | Rf-Werte (Methylenchlorid/Methanol 1 : 2) |
|---|---|---|
| a) | 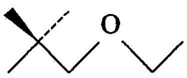 | 0.38 |
| b) | 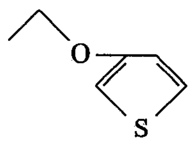 | 0.40 |
| c) | 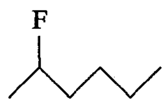 | 0.34 |
| d) | 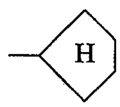 | 0.36 |
| e) | 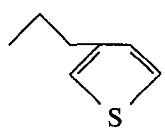 | 0.37 |
| f) | 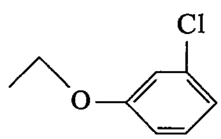 | 0.38 |

## Beispiel 23

2-Aza-3-(4-carboxyethylanilino)-6-(3-(R,S)-biphenylcarbonyloxy-1-octenyl)-7-biphenylcarbonyloxybicyclo[3.3.0]octen-2

77 mg Thiolactimether (0.12 mmol) (Beispiel 21) werden mit 19.8 mg 4-Aminobenzoesäureethylester (0.12 mmol) in 1 ml Methanol und 1 Tropfen Eisessig 3 Stunden unter Rückfluß gekocht. Das Lösungsmittel wird abgezogen und der Rückstand an Kieselgel (Laufmittel: Methylenchlorid/Methanol 8 : 1) chromatographiert.

Ausbeute:
87.6 mg (96.2%)
NMR ($CDCl_3$):
$\delta$ ppm: 6.8—8.1, m, 22H (Aryl-H), 6.0—6.4, m, 1H (N=C—N—H), 5.5—5.7, m, 2H (H—C=C—H), 4.7—5.5, m, 2H (O=C—O—C—H), 4.0—4.3, m, 1H (N—C=N—C—H), 4.25, q, 2H ($CO_2$—$CH_3$—), 1.3, t, 3H ($CO_2CH_2CH_3$)
IR (Film):
$cm^{-1}$: 3400—3000 (breit, NH Amidin), 1700 (C=O Ester), 1640, 1590 (Amidin)
Rf (Methanol/Methylenchlorid 1 : 8):
0.11

In Analogie zu Beispiel 23 lassen sich auch aus den Verbindungen der Beispiele 21a—21f die Amidine XXIV

($R^4$, $R^5$ = Biphenylcarbonyl, Z = $CO_2C_2H_5$, Y = 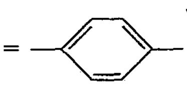)

herstellen.

| Beispiel 23 | $R^2$ | Rf-Wert (Methylenchlorid/Methanol 8 : 1) |
|---|---|---|
| a) | 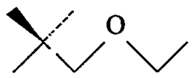 | 0.13 |
| b) | 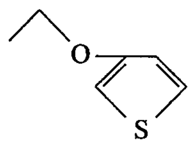 | 0.15 |
| c) | 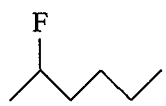 | 0.14 |
| d) | 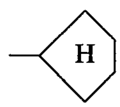 | 0.13 |
| e) | 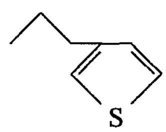 | 0.15 |
| f) | 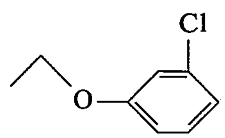 | 0.12 |

## Beispiel 24

2-Aza-3-(1-aza-4-carboxybutyl-1)-6-(3-(R,S)-hydroxy-1-octenyl-1-octenyl)-7-hydroxybicyclo[3.3.0]octen-2

71 mg Diester (0,1 mmol) (Beispiel 22) werden mit 12,8 mg Kaliumcarbonat (0.1 mmol) in 1 ml absolutem Methanol 2 Stunden bei Raumtemperatur gerührt. Nach Neutralisation mit Salzsäure wird mit 15 ml Essigester versetzt, die org. Phase abgetrennt und mit Wasser gewaschen. Die wäßrige Phase wird mit Kochsalz gesättigt und mehrfach mit Essigester extrahiert. Die vereinigten org. Phasen werden über $MgSO_4$ getrocknet und nach Filtration von Trockenmittel eingeengt. Der Rückstand wird an Kieselgel (Laufmittel: Methylenchlorid/Methanol 1 : 1) chromatographiert.

Ausbeute:
25.3 mg (72.2%)
NMR ($CDCl_3$):
$\delta$ ppm: 5.45—5.7, m, 2H (H—C=C—H), 4.2—4.4, m, 1H (N—C=N—C—H), 3.6—4.2, m, 2H (CH—OH)
IR (Film):
$cm^{-1}$: 2500—3500 (breit, $CO_2H$, CH, NH), 1685, 1560 (Amidin), 1560, 1410 (Carboxylat)
Rf (Methylenchlorid/Methanol 1 : 2):
0.20 ($\beta$-Isomeres)
0.17 ($\alpha$-Isomeres)

In Analogie zu Beispiel 24 lassen sich auch die Verbindungen der Beispiele 22a—22f zu Amidinen der Formel I (X = NH, Y = $-(CH_2)_3-$, Z = $CO_2H$) verseifen.

| Beispiel 24 | $R^2$ | Rf-Wert ($\beta/\alpha$) (Methylenchlorid/Methanol 1:2) |
|---|---|---|
| a) | 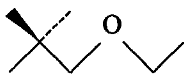 | 0.21/0.19 |
| b) | 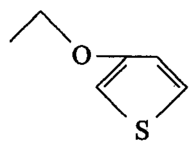 | 0.23/0.20 |
| c) | 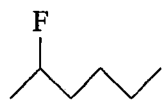 | 0.22/0.20 |
| d) | 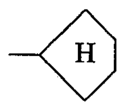 | 0.21/0.20 |
| e) | 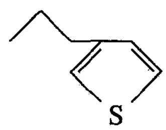 | 0.22/0.20 |
| f) | 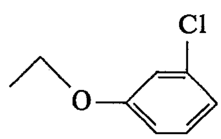 | 0.20/0.18 |

## Beispiel 25

2-Aza-3-(4-carboxymethylanilino)-6-(3-(R,S)-hydroxy-1-octenyl)-7-hydroxy-bicyclo[3.3.0]octen-2

79 mg Diester (Beispiel 23) (0.11 mmol) werden mit 14.1 mg Kaliumcarbonat (0.11 mmol) in 1 ml trockenem Methanol 2 Stunden bei Raumtemperatur gerührt. Nach Neutralisation mit Salzsäure wird mit 15 ml Essigester versetzt, die org. Phase abgetrennt und mit Wasser gewaschen. Die wäßrige Phase wird mit Kochsalz gesättigt und mehrfach mit Essigester extrahiert. Die vereinigten org. Phasen werden über $MgSO_4$ getrocknet und nach Filtration eingeengt. Der Rückstand wird an Kieselgel (Laufmittel: Methylenchlorid/Methanol 3 : 1) chromatographiert.

Ausbeute:
33.3 mg (75.4%)
NMR ($CDCl_3$):
$\delta$ ppm: 7.8, 6.9, d (J = 8Hz), 4H (Aryl-H), 6.0–6.4, m, 1H (N=C–N–H), 5.5–5.7, m, 2H (H–C=C–H), 4.2–4.4, m, 1H (N–C=N–C–H), 3.6–4.2, m, 2H (CH–OH), 3.7, s, 3H ($CO_2CH_3$)
IR (Film):
$cm^{-1}$: 3600–3000 (breit OH, NH Amidin), 1700 (C=O Ester), 1640, 1590 (Amidin)
Rf (Methylenchlorid/Methanol 3 : 1):
0.22 ($\beta$-Isomeres)
0.19 ($\alpha$-Isomeres)

In Analogie zu Beispiel 25 lassen sich auch die Verbindungen der Beispiele 23a–23f zu Amidinen der Formel I

(X = NH, Y = 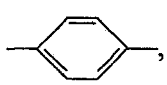, Z = $CO_2CH_3$)

verseifen.

| Beispiel 25 | $R^2$ | Rf-Wert ($\beta/\alpha$) (Methylenchlorid/Methanol 3 : 1) |
|---|---|---|
| a) | 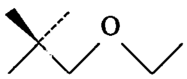 | 0.24/0.21 |
| b) | 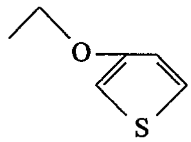 | 0.22/0.20 |
| c) | 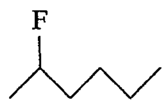 | 0.23/0.20 |
| d) | 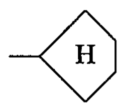 | 0.22/0.20 |
| e) | 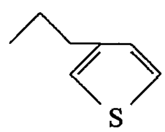 | 0.23/0.20 |
| f) | 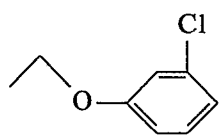 | 0.21/0.20 |

## Beispiel 26

**2-Aza-3-(4-hydroxybutylamino)-6-(3-(R,S)-biphenylcarbonyloxy-1-octenyl)-7-biphenylcarbonyloxybicyclo[3.3.0]octen-2**

105 mg Thiolactimether (0.16 mmol) (Beispiel 21) werden mit 14.6 mg 4-Aminobutanol (0.16 mmol) und 3 Tropfen Eisessig in 1.5 ml Methanol 5 Stunden unter Rückfluß gekocht. Das Lösungsmittel wird abgezogen und der Rückstand an Kieselgel (Laufmittel: Methylenchlorid/Methanol 3 : 1) chromatographiert.

Ausbeute:
94 mg (84.2%)
NMR ($CDCl_3$):
$\delta$ ppm: 7.6–8.1, m, 18H (Aryl-H), 5.5–5.7, m, 3H (H–C=C–H, N=C–NH), 4.7–5.5, m, 2H (O=C–O–C–H), 4.35, m, 1H (N–C=N–C–H)
IR (Film):
$cm^{-1}$: 3600–3000 (breit, OH, NH), 1760 (C=O Ester),
Rf (Methylenchlorid/Methanol 1 : 3):
0.08

In Analogie zu Beispiel 26 lassen sich aus den Verbindungen der Beispiele 21a–21f die Amidine XXIV ($Z = CH_2OH$, $R^4$, $R^5$ = Biphenylcarbonyl, $Y = -(CH_2)-_3$) herstellen.

| Beispiel 26 | $R^2$ | Rf-Werte (Methylenchlorid/Methanol 1 : 3) |
|---|---|---|
| a) | 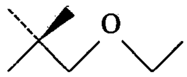 | 0.10 |
| b) | 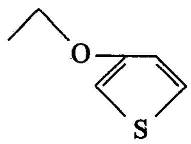 | 0.09 |
| c) | 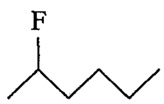 | 0.11 |
| d) | 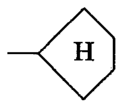 | 0.11 |
| e) | 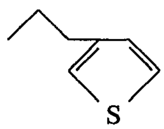 | 0.09 |
| f) | 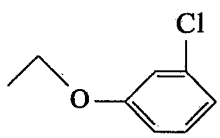 | 0.10 |

## Beispiel 27

2-Aza-3-(4-hydroxybutylamino)-6-(3-(R,S)-hydroxy-1-octenyl)-7-hydroxybicyclo[3.3.0]octen-2

90 mg Diester (0.129 mmol) (Beispiel 26) werden mit 16.5 mg $K_2CO_3$ (0.129 mmol) in 1.3 ml absolutem Methanol 2 Stunden bei Raumtemperatur gerührt. Nach Neutralisation mit Salzsäure wird die Lösung am Rotavapor eingeengt. Der Rückstand wird mehrmals mit Essigester ausgekocht, das Lösungsmittel abgezogen und der Rückstand an Kieselgel (Laufmittel: Methylenchlorid/Methanol 1 : 1) chromatographiert.

Ausbeute:
31.9 mg (73.4%)
NMR ($CDCl_3$):
$\delta$ ppm: 5.45–5.7, m, 2H (H–C=C–H), 4.2–4.4, m, 1H (N–C=N–C–H), 3.55–4.2, m, 4H (CH–OH)
IR (Film):
$cm^{-1}$: 3600–3000 (breit, OH, NH)
Rf (Methylenchlorid/Methanol 1 : 5):
0.12

In Analogie zu Beispiel 27 lassen sich auch die Verbindungen der Beispiele 26a–26f zu Amidinen der allgemeinen Formel I ($Z = CH_2OH$, $Y = -(CH_2)_3-$) verseifen.

| Beispiel 27 | $R^2$ | Rf-Werte (Methylenchlorid/Methanol 1 : 1) |
|---|---|---|
| a) | 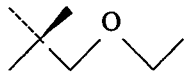 | 0.11 |
| b) | 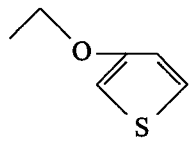 | 0.13 |
| c) | 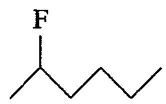 | 0.13 |
| d) | 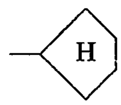 | 0.14 |
| e) | 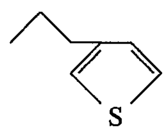 | 0.10 |
| f) | 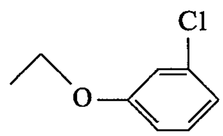 | 0.13 |

## Beispiel 28

### 2-Aza-3-(4-dimethylaminobutylamino)-6-(3-(R,S)-biphenylcarbonyloxy-1-octenyl)-7-biphenylcarbonyloxybicyclo[3.3.0]octen-2

98 mg Thiolactimether (0.15 mmol) (Beispiel 21) werden mit 13.5 mg Dimethylaminobutylamin (0.15 mmol) und 3 Tropfen Eisessig in 5 ml Methanol 4 Stunden unter Rückfluß erhitzt. Das Lösungsmittel wird abgezogen und der Rückstand an Kieselgel (Laufmittel: Methylenchlorid/Methanol 3 : 1) chromatographiert.

Ausbeute:
92.8 mg (89.3%)
NMR ($CDCl_3$):
$\delta$ ppm: 7.6–8.1, m, 18H (Aryl-H), 5.5–5.7, m, 3H (H–C=C–H, N=C–NH), 4.7–5.5, m, 2H (O=C–O–C–H), 4.3, m, 1H (N–C=N–C–H), 2.2, s, 6H ($N–CH_3$)
IR (Film):
$cm^{-1}$: 3200 (breit, NH), 1710 (C=O Arylester)
Rf (Methylenchlorid/Methanol 1 : 3):
0.28

In Analogie zu Beispiel 28 lassen sich aus den Verbindungen der Beispiele 21a–21f die Amidine XXIV ($Z = CH_2–N(CH_3)_2$, $R^4$, $R^5$ = Biphenylcarbonyl, $Y = –(CH_2)_3–$) herstellen.

| Beispiel 28 | $R^2$ | Rf-Werte (Methylenchlorid/Methanol 1:3) |
|---|---|---|
| a) | 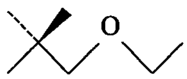 | 0.25 |
| b) | 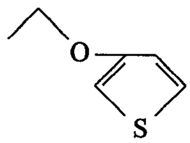 | 0.23 |
| c) | 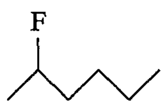 | 0.26 |
| d) | 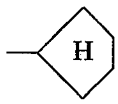 | 0.27 |
| e) | 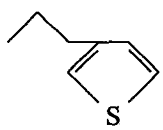 | 0.24 |
| f) | 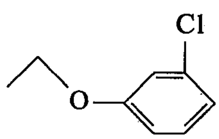 | 0.26 |

## Beispiel 29

### 2-Aza-3-(4-dimethylaminobutylamino)-6-(3-(R,S)-hydroxy-1-octenyl)-7-hydroxybicyclo[3.3.0]octen-2

90 mg Diester (0.129 mmol) (Beispiel 28) werden mit 16.5 mg Kaliumcarbonat (0.129 mmol) in 1.4 ml absolutem Methanol 2 Stunden bei Raumtemperatur gerührt. Nach Neutralisation mit Salzsäure wird das Lösungsmittel abgezogen und der Rückstand mehrfach mit Essigester ausgekocht. Nach Abziehen des Lösungsmittels wird der Rückstand an Kieselgel (Laufmittel: Methylenchlorid/Methanol 1 : 1) chromatographiert.

Ausbeute:
32,4 mg (74.1%).
NMR ($CDCl_3$):
$\delta$ ppm: 7.1, s (breit), 1H (NH), 5.5—5.7, m, 2H (H—C=C—H), 4.3, m, 1H (N—C=N—C—H), 2.2, s, 6H ($NCH_3$)
IR (Film):
$cm^{-1}$: 3200 (breit, NH)
Rf (Methylenchlorid/Methanol 1 : 3):
0.11

In Analogie zu Beispiel 29 lassen sich auch die Verbindungen der Beispiele 28a—28f zu Amidinen der Formel I ($Z = CH_2—N(CH_3)_2$, $Y = —(CH_2)_3—$) verseifen.

| Beispiel 29 | $R^2$ | Rf-Werte (Methylenchlorid/Methanol 1:3) |
|---|---|---|
| a) | 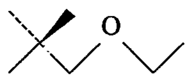 | 0.12 |
| b) | 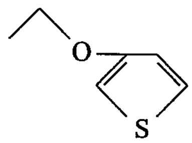 | 0.14 |
| c) | 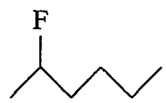 | 0.13 |
| d) | 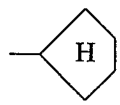 | 0.13 |
| e) | 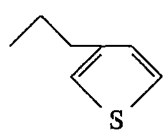 | 0.12 |
| f) | 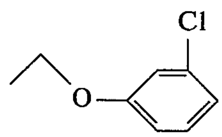 | 0.10 |

**Patentansprüche für die Vertragsstaaten: BE, CH, DE, FR, GB, IT, LI, NL, SE**

1. Verbindungen der Formel I

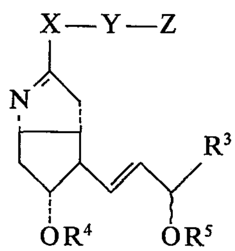

(I)

in welcher bedeuten

X ein Sauerstoff- oder Schwefelatom oder eine NH-Gruppe,

Y einen geradkettigen oder verzweigten Alkylenrest mit bis zu acht Kohlenstoffatomen oder einen geradkettigen oder verzweigten ungesättigten aliphatischen Rest mit drei bis acht Kohlenstoffatomen oder einen cycloaliphatischen Rest mit drei bis sechs Kohlenstoffatomen oder einen Phenylenrest,

Z einen Rest der Formel $-CO_2R^1$, $-CH_2OH$ oder $CH_2-N(R^2)_2$,
wobei bedeuten:

$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit drei bis sechs Kohlenwasserstoffatomen oder einen cycloaliphatischen Kohlenwasserstoffrest mit drei bis sieben Kohlenstoffatomen oder einen araliphatischen Kohlenwasserstoffrest mit sieben bis neun Kohlenstoffatomen, oder ein physiologisch verträgliches Metall-, $NH_4$- oder ein Ammoniumion in Form des 5,5-Dimethyl-Dicyclohexyl- oder Tris(hydroxymethyl)methylammoniums

$R^2$ Wasserstoff oder einen geradkettigen oder verzweigten aliphatischen Kohlenwasserstoffrest mit bis zu fünf C-Atomen oder $R^2-R^2$ zusammen auch eine $-(CH_2)_n$-Gruppe, mit $n = 3-6$

$R^3$ einen unsubstituierten Phenylrest, oder einen Phenyl-, Thienyl- oder Furylrest die im Kern 1—3fach substituiert sein können mit Halogen, Trifluormethyl, und/oder Alkyl oder Alkoxy mit je 1—6 C-Atomen,
oder einen cycloaliphatischen Rest mit 3—8 Kohlenstoffatomen oder
einen geradkettigen oder verzweigten Alkylrest bis zu acht Kohlenstoffatomen oder einen geradkettigen oder verzweigten ungesättigten aliphatischen Kohlenwasserstoffrest mit drei bis acht Kohlenstoffatomen, die ihrerseits substituiert sein können mit
a) einem geradkettigen oder verzweigten Alkoxyrest mit bis zu sechs Kohlenstoffatomen oder einem geradkettigen oder verzweigten Alkenyloxy- oder Alkinyloxyrest mit drei bis sechs Kohlenstoffatomen,
b) Halogen, Phenyl oder einem $\alpha$- oder $\beta$-Thienyl- oder $\alpha$- oder $\beta$-Furylrest, die ihrerseits im Kern 1—3fach substituiert sein können mit Halogen, Trifluormethyl, und/oder Alkyl oder Alkoxy mit je 1—6 C-Atomen,
c) einem Phenoxy-, einem $\alpha$-oder $\beta$-Thienyloxyrest oder einem Cycloalkoxyrest mit 3—7 Kohlenstoffatomen, wobei die genannten Reste ihrerseits im Kern 1—3fach substituiert sein können mit Halogen, Trifluormethyl und/oder Alkyl oder Alkyloxy mit je 1—6 C-Atomen,
$R^4$, $R^5$ jeweils Wasserstoff.

2. Verfahren zur Herstellung von Verbindungen der Formel I

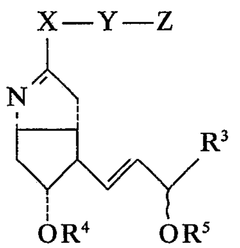

(I)

in welcher bedeuten

X ein Sauerstoff- oder Schwefelatom oder eine NH-Gruppe,
Y einen geradkettigen oder verzweigten Alkylenrest mit bis zu acht Kohlenstoffatomen oder einen geradkettigen oder verzweigten ungesättigten aliphatischen Rest mit drei bis acht Kohlenstoffatomen oder einen cycloaliphatischen Rest mit drei bis sechs Kohlenstoffatomen oder einen Phenylenrest,
Z einen Rest der Formel $-CO_2R^1$, $-CH_2-OH$ oder $CH_2N(R^2)_2$
wobei bedeuten
$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit drei bis sechs Kohlenwasserstoffatomen oder einen cycloaliphatischen Kohlenwasserstoffrest mit drei bis sieben Kohlenstoffatomen oder einen araliphatischen Kohlenwasserstoffrest mit sieben bis neun Kohlenstoffatomen, oder ein physiologisch verträgliches Metall-, $NH_4$- oder ein Ammoniumion in Form des 5,5-Dimethyl-Dicyclohexyl- oder Tris(hydroxymethyl)methylammoniums
$R^2$ Wasserstoff oder einen geradkettigen oder verzweigten aliphatischen Kohlenwasserstoffrest bis zu fünf C-Atomen oder $R^2$—$R^2$ zusammen auch eine $-(CH_2)_n$-Gruppe, mit $n = 3-6$
$R^3$ einen unsubstituierten Phenylrest, oder einen Phenyl-, Thienyl- oder Furylrest der im Kern 1—3fach substituiert sein kann mit Halogen, Trifluormethyl, und/oder Alkyl oder Alkoxy mit je 1—6 C-Atomen,
oder einen cycloaliphatischen Rest mit 3—8 Kohlenstoffatomen oder
einen geradkettigen oder verzweigten Alkylrest mit bis zu acht Kohlenstoffatomen oder einen geradkettigen oder verzweigten ungesättigten aliphatischen Kohlenwasserstoffrest mit drei bis acht Kohlenstoffatomen, die ihrerseits substituiert sein können mit
a) einem geradkettigen oder verzweigten Alkoxyrest mit bis zu sechs Kohlenstoffatomen oder einem geradkettigen oder verzweigten Alkenyloxy- oder Alkinyloxyrest mit drei bis sechs Kohlenstoffatomen,
b) Halogen, Phenyl oder einem $\alpha$- oder $\beta$-Thienyl- oder $\alpha$- oder $\beta$-Furylrest, die ihrerseits im Kern 1—3fach substituiert sein können mit Halogen, Trifluormethyl, und/oder Alkyl oder Alkoxy mit je 1—6 C-Atomen,
c) einem Phenoxy-, einem $\alpha$- oder $\beta$-Thienyloxyrest oder einem Cycloalkoxyrest mit 3—7 Kohlenstoffatomen, wobei die genannten Reste ihrerseits im Kern 1—3fach substituiert sein können mit Halogen, Trifluormethyl und/oder Alkyl oder Alkyloxy mit 1—6 C-Atomen,
$R^4$, $R^5$ jeweils Wasserstoff,

dadurch gekennzeichnet, daß man

a) eine Verbindung der Formel XVII oder XVIII

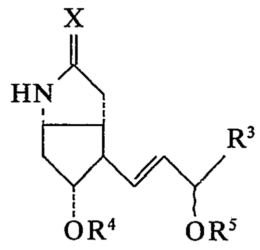

(XVII/XVIII)

worin $R^3$ die oben genannte Bedeutung hat, $R^4$ und $R^5$ gleich oder verschieden sind und eine unter neutralen oder basischen Bedingungen leicht abspaltbare Schutzgruppe darstellen und X ein Sauerstoff- oder Schwefelatom bedeutet, mit einem Alkylhalogenid der Formel IXX

$$Z - Y - Hal \qquad (IXX)$$

worin Hal Jod, Chlor oder Brom bedeutet und Y und Z die oben genannten Bedeutungen haben, alkyliert, oder

b) eine Verbindung der Formel XXII

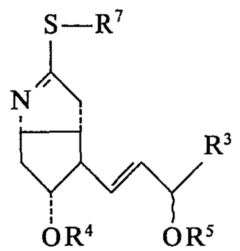

(XXII)

worin $R^3$, $R^4$ und $R^5$ die oben genannten Bedeutungen haben und $R^7$ einen $C_{1-4}$-Alkylrest bedeutet, mit einem Amin der Formel

$$Z - Y - NH_2 \qquad (XXIII)$$

worin Z und Y die oben genannten Bedeutungen haben, umsetzt,

c) gegebenenfalls in einer Verbindung der Formel I die Schutzgruppen $R^4$ und $R^5$ durch basisch katalysierte Hydrolyse abspaltet,

d) gegebenenfalls in einer Verbindung der Formel I, in welcher Z den Rest $CO_2R^1$ bedeutet, wobei $R^1$ den oben genannten Rest darstellt und $R^4$ und $R^5$ Wasserstoff bedeuten, zu einer Verbindung der Formel I verseift, in welcher Z den Rest $CO_2R^1$ darstellt, wobei $R^1$ Wasserstoff oder ein physiologisch verträgliches Kation bedeutet,

e) gegebenenfalls eine Verbindung der Formel I, in welcher Z den Rest $CO_2R^1$ darstellt, wobei $R^1$ Wasserstoff oder ein Kation bedeutet, zu einer Verbindung der Formel I verestert, worin Z den Rest $CO_2R^1$ darstellt, wobei $R^1$ die zur Formel I genannten Bedeutungen hat, jedoch nicht Wasserstoff oder ein Kation bedeutet, und

f) gegebenenfalls in einer Verbindung der Formel I, in welcher Z den Rest $CO_2R^1$ darstellt, wobei $R^1$ ein physiologisch verträgliches Kation darstellt, dieses Kation gegen ein anderes austauscht.

3. Verfahren zur Herstellung von Arzneimitteln, dadurch gekennzeichnet, daß man eine Verbindung der Formel I nach Anspruch 1, gegebenenfalls mit üblichen pharmazeutischen Trägern und/oder Stabilisatoren, in eine therapeutisch geeignete Darreichungsform bringt.

4. Arzneimittel, gekennzeichnet durch den Gehalt an einer Verbindung der Formel I nach Anspruch 1 in Mischung mit einem pharmazeutisch üblichen Träger und/oder Stabilisator.

5. Verbindung der Formel I nach Anspruch 1 zur Verwendung als Arzneimittel.

**Patentanspruch für den Vertragsstaat: AT**

Verfahren zur Herstellung von Verbindungen der Formel I

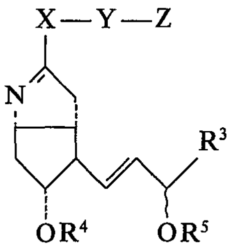

(I)

in welcher bedeuten

X ein Sauerstoff- oder Schwefelatom oder eine NH-Gruppe,

Y einen geradkettigen oder verzweigten Alkylenrest mit bis zu acht Kohlenstoffatomen oder einen geradkettigen oder verzweigten ungesättigten aliphatischen Rest mit drei bis acht Kohlenstoffatomen oder einen cycloaliphatischen Rest mit drei bis sechs Kohlenstoffatomen oder einen Phenylenrest,

Z einen Rest der Formel $-CO_2R^1$, $-CH_2-OH$ oder $CH_2N(R^2)_2$ wobei bedeuten

$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit drei bis sechs Kohlenwasserstoffatomen oder einen cycloaliphatischen Kohlenwasserstoffrest mit drei bis sieben Kohlenstoffatomen oder einen araliphatischen Kohlenwasserstoffrest mit sieben bis neun Kohlenstoffatomen, oder ein physiologisch verträgliches Metall-, $NH_4$- oder ein Ammoniumion in der Form des 5,5-Dimethyl-Dicyclohexyl- oder Tris-(hydroxymethyl)methylammoniums

$R^2$ Wasserstoff oder einen geradkettigen oder verzweigten aliphatischen Kohlenwasserstoffrest bis zu fünf C-Atomen oder $R^2-R^2$ zusammen auch eine $-(CH_2)_n$-Gruppe, mit $n = 3-6$

$R^3$ einen unsubstituierten Phenylrest, oder einen Phenyl-, Thienyl- oder Furylrest, der im Kern 1—3fach substituiert sein kann mit Halogen, Trifluormethyl, und/oder Alkyl oder Alkoxy mit je 1—6 C-Atomen,
oder einen cycloaliphatischen Rest mit 3—8 Kohlenstoffatomen oder
einen geradkettigen oder verzweigten Alkylrest mit bis zu acht Kohlenstoffatomen oder einen geradkettigen oder verzweigten ungesättigten aliphatischen Kohlenwasserstoffrest mit drei bis acht Kohlenstoffatomen, die ihrerseits substituiert sein können mit

a) einem geradkettigen oder verzweigten Alkoxyrest mit bis zu sechs Kohlenstoffatomen oder einem geradkettigen oder verzweigten Alkenyloxy- oder Alkinyloxyrest mit drei bis sechs Kohlenstoffatomen,
b) Halogen, Phenyl oder einem $\alpha$- oder $\beta$-Thienyl- oder $\alpha$- oder $\beta$-Furylrest, die ihrerseits im Kern 1—3fach substituiert sein können mit Halogen, Trifluormethyl, und/oder Alkyl oder Alkoxy mit je 1—6 C-Atomen,
c) einem Phenoxy-, einem $\alpha$- oder $\beta$-Thienyloxyrest oder einem Cycloalkoxyrest mit 3—7 Kohlenstoffatomen, wobei die genannten Reste ihrerseits im Kern 1—3fach substituiert sein können mit Halogen, Trifluormethyl und/oder Alkyl oder Alkyloxy mit 1—6 C-Atomen,

$R^4$, $R^5$ jeweils Wasserstoff,

dadurch gekennzeichnet, daß man

a) eine Verbindung der Formel XVII oder XVIII

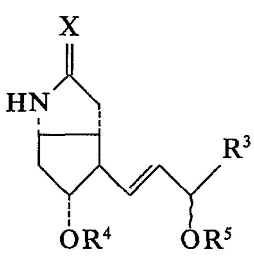

(XVII/XVIII)

worin $R^3$ die oben genannte Bedeutung hat, $R^4$ und $R^5$ gleich oder verschieden sind und eine unter neutralen oder basischen Bedingungen leicht abspaltbare Schutzgruppe darstellen und X ein Sauerstoff- oder Schwefelatom bedeutet, mit einem Alkylhalogenid der Formel IXX

Z – Y – Hal (IXX)

worin Hal Jod, Chlor oder Brom bedeutet und Y und Z die oben genannten Bedeutungen haben, alkyliert, oder

b) eine Verbindung der Formel XXII

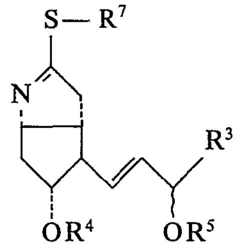

(XXII)

worin $R^3$, $R^4$ und $R^5$ die oben genannten Bedeutungen haben und $R^7$ einen $C_{1-4}$-Alkylrest bedeutet, mit einem Amin der Formel

$Z – Y – NH_2$ (XXIII)

worin Z und Y die oben genannten Bedeutungen haben, umsetzt,

c) gegebenenfalls in einer Verbindung der Formel I die Schutzgruppen $R^4$ und $R^5$ durch basisch katalysierte Hydrolyse abspaltet,

d) gegebenenfalls in einer Verbindung der Formel I, in welcher Z den Rest $CO_2R^1$ bedeutet, wobei $R^1$ den oben genannten Rest darstellt und $R^4$ und $R^5$ Wasserstoff bedeuten, zu einer Verbindung der Formel I verseift, in welcher Z den Rest $CO_2R^1$ darstellt, wobei $R^1$ Wasserstoff oder ein physiologisch verträgliches Kation bedeutet,

e) gegebenenfalls eine Verbindung der Formel I, in welcher Z den Rest $CO_2R^1$ darstellt, wobei $R^1$ Wasserstoff oder ein Kation bedeutet, zu einer Verbindung der Formel I verestert, worin Z den Rest $CO_2R^1$ darstellt, wobei $R^1$ die zur Formel I genannten Bedeutungen hat, jedoch nicht Wasserstoff oder ein Kation bedeutet, und

f) gegebenenfalls in einer Verbindung der Formel I, in welcher Z den Rest $CO_2R^1$ darstellt, wobei $R^1$ ein physiologisch verträgliches Kation darstellt, dieses Kation gegen ein anderes austauscht.

**Claims for the Contracting States: BE, CH, DE, FR, GB, IT, LI, NL, SE**

1. A compound of the formula I

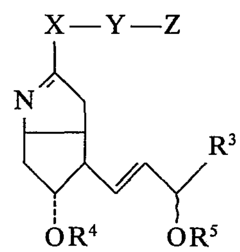

(I)

in which

X is an oxygen or sulfur atom or an NH group,

Y is a straight-chain or branched alkylene radical having up to eight carbon atoms, a straight-chain or branched unsaturated aliphatic radical having three to eight carbon atoms, a cycloaliphatic radical having three to six carbon atoms or a phenylene radical,

Z is a radical of the formula $-CO_2R^1$, $-CH_2OH$ or $CH_2N(R^2)_2$,

wherein

$R^1$ is hydrogen, a straight-chain or branched alkyl radical having up to 8 carbon atoms, a straight-chain or branched unsaturated aliphatic hydrocarbon radical having three to six carbon atoms, a cycloaliphatic hydrocarbon radical having three to seven carbon atoms, an araliphatic hydrocarbon radical having seven to nine carbon atoms or a physiologically acceptable metal ion, $HH_4$ ion or an ammonium ion in the form of the 5,5-dimethyl-dicyclohexyl- or trishydroymethyl-methylammonium

$R^2$ is hydrogen or a straight-chain or branched aliphatic hydrocarbon radical having up to five C atoms, or $R^2$—$R^2$ conjointly are a $-(CH_2)_n$ group with $n = 3–6$,

$R^3$ is an unsubstituted phenyl radical or a phenyl, thienyl or furyl radical, which can be monosubstituted to trisubstituted in the nucleus by halogen, trifluoromethyl and/or alkyl or alkoxy each having 1—6 C atoms,
or a cycloaliphatic radical having 3—8 carbon atoms or
a straight-chain or branched alkyl radical having up to eight carbon atoms or a straight-chain or branched unsaturated aliphatic hydrocarbon radical having three to eight carbon atoms, which radicals can in turn be substituted by

a) a straight-chain or branched alkoxy radical having up to six carbon atoms or a straight-chain or branched alkenyloxy or alkynyloxy radical having three to six carbon atoms,
b) halogen, phenyl or an $\alpha$- or $\beta$-thienyl or $\alpha$- or $\beta$-furyl radical which in turn can be monosubstituted to trisubstituted in the nucleus by halogen, trifluoromethyl and/or alkyl or alkoxy each having 1—6 C atoms, or
c) a phenoxy radical, an $\alpha$- or $\beta$-thienyloxy radical or a cycloalkoxy radical having 3—7 carbon atoms, it being possible for the said radicals to be in turn monosubstituted to trisubstituted in the nucleus by halogen, trifluoromythyl and/or alkyl or alkoxy having 1—6 C atoms, and

$R^4$ and $R^5$ are each hydrogen.

2. A process for the manufacture of compounds of the formula I

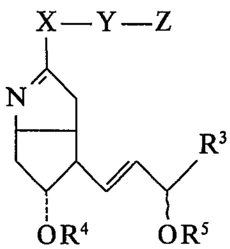

(I)

in which

X is an oxygen or sulfur atom or an NH group,
Y is a straight-chain or branched alkylene radical having up to eight carbon atoms, a straight-chain or branched unsaturated aliphatic radical having three to eight carbon atoms, a cycloaliphatic radical having three to six carbon atoms or a phenylene radical,
Z is a radical of the formula $-CO_2R^1$, $-CH_2OH$ or $CH_2N(R^2)_2$,
wherein
$R^1$ is hydrogen, a straight-chain or branched alkyl radical having up to 8 carbon atoms, a straight-chain or branched unsaturated aliphatic hydrocarbon radical having three to six carbon atoms, a cycloaliphatic hydrocarbon radical having three to seven carbon atoms, an araliphatic hydrocarbon radical having seven to nine carbon atoms or a physiologically acceptable metal ion, $NH_4$ ion or an ammonium ion in the form of the 5,5-dimethyl-dicyclohexyl- or tris(hydroxymethyl)methyl ammonium,
$R^2$ is hydrogen or a straight-chain or branched aliphatic hydrocarbon radical having up to five C atoms, or $R^2$—$R^2$ conjointly are a $-(CH_2)_n$ group with $n = 3$—6,
$R^3$ is an unsubstituted phenyl radical or a phenyl, thienyl or furyl radical which can be monosubstituted to trisubstituted in the nucleus by halogen, trifluoromethyl and/or alkyl or alkoxy each having 1—6 C atoms,
or a cycloaliphatic radical having 3—8 carbon atoms or
a straight-chain or branched alkyl radical having up to eight carbon atoms or a straight-chain or branched unsaturated aliphatic hydrocarbon radical having three to eight carbon atoms, which radicals can in turn be substituted by

a) a straight-chain or branched alkoxy radical having up to six carbon atoms or a straight-chain or branched alkenyloxy or alkynyloxy radical having three to six carbon atoms,
b) halogen, phenyl or an $\alpha$- or $\beta$-thienyl or $\alpha$- or $\beta$-furyl radical which in turn can be monosubstituted to trisubstituted in the nucleus by halogen, trifluoromethyl and/or alkyl or alkoxy each having 1—6 C atoms, or
c) a phenoxy radical, an $\alpha$- or $\beta$-thienyloxy radical or a cycloalkoxy radical having 3—7 carbon atoms, it being possible for the said radicals to be in turn monosubstituted to trisubstituted in the nucleus by halogen, trifluoromythyl and/or alkyl or alkoxy having 1—6 C atoms, and

$R^4$ and $R^5$ are each hydrogen which comprises

a) alkylating a compound of the formula XVII or XVIII

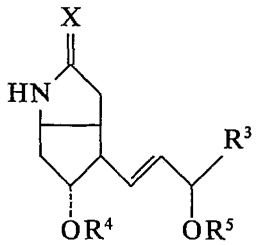

(XVII/XVIII)

wherein $R^3$ has the meaning given above, $R^4$ and $R^5$ are identical or different and represent a protective group which can readily be eliminated under neutral or basic conditions, and X is an oxygen or sulfur atom, with an alkyl halide of the formula IXX

$$Z - Y - Hal \quad \text{(IXX)}$$

wherein Hal is iodine, chlorine or bromine and Y and Z have the meanings given above, or

b) reacting a compound of the formula XXII

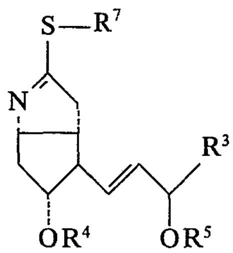

(XXII)

wherein $R^3$, $R^4$ and $R^5$ have the meanings given above and $R^7$ is a $C_{1-4}$-alkyl radical, with an amine of the formula

$$Z - Y - NH_2 \quad \text{(XXIII)}$$

wherein Z and Y have the meanings given above,

c) if appropriate, eliminating the protective groups $R^4$ and $R^5$ in a compound of the formula I by a hydrolysis with basic catalysis,

d) if appropriate, saponifying a compound of the formula I, in which Z is the radical $CO_2R^1$, $R^1$ representing the abovementioned radical and $R^4$ and $R^5$ being hydrogen, to give a compound of the formula I, in which Z represents the radical $CO_2R^1$, $R^1$ being hydrogen or a physiologically acceptable cation,

e) if appropriate, esterifying a compound of the formula I, in which Z represents the radical $CO_2R^1$, $R^1$ being hydrogen or a cation, to give a compound of the formula I, wherein Z represents the radical $CO_2R^1$, $R^1$ having the meanings mentioned under the formula I, but with the exclusion of hydrogen or a cation, and

f) if appropriate, in a compound of the formula I, in which Z represents the radical $CO_2R^1$, $R^1$ being a physiologically acceptable cation, exchanging this cation for another cation.

3. A process for the manufacture of medicaments, which comprises converting a compound of the formula I, as claimed in claim 1 if appropriate together with customary pharmaceutical excipients and/or stabilizers, into a therapeutically suitable administration form.

4. A medicament, which comprises a compound of the formula I as claimed in claim 1 in a mixture with a pharmaceutically customary excipient and/or stabilizer.

5. A compound of the formula I as claimed in claim 1 for use as a medicament.

**Claim for the Contracting State: AT**

A process for the manufacture of compounds of the formula I

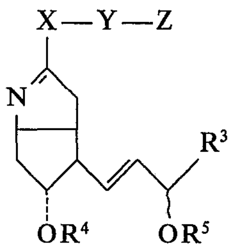

(I)

in which

X is an oxygen or sulfur atom or an NH group,

Y is a straight-chain or branched alkylene radical having up to eight carbon atoms, a straight-chain or branched unsaturated aliphatic radical having three to eight carbon atoms, a cycloaliphatic radical having three to six carbon atoms or a phenylene radical,

Z is a radical of the formula $-CO_2R^1$, $-CH_2OH$ or $CH_2N(R^2)_2$, wherein

$R^1$ is hydrogen, a straight-chain or branched alkyl radical having up to 8 carbon atoms, a straight-chain or branched unsaturated aliphatic hydrocarbon radical having three to six carbon atoms, a cycloaliphatic hydrocarbon radical having three to seven carbon atoms, an araliphatic hydrocarbon radical having seven to nine carbon atoms or a physiologically acceptable metal ion, $NH_4$ ion or an ammonium ion in the form of the 5,5-dimethyl-dicyclohexyl- or tris(hydroxymethyl)methylammonium,

$R^2$ is hydrogen or a straight-chain or branched aliphatic hydrocarbon radical having up to five C atoms, or $R^2$—$R^2$ conjointly are a $-(CH_2)_n$ group with $n = 3–6$,

$R^3$ is an unsubstituted phenyl radical or a phenyl, thienyl or furyl radical which can be monosubstituted to trisubstituted in the nucleus by halogen, trifluoromethyl and/or alkyl or alkoxy each having 1–6 C atoms,
or a cycloaliphatic radical having 3–8 carbon atoms or
a straight-chain or branched alkyl radical having up to eight carbon atoms or a straight-chain or branched unsaturated aliphatic hydrocarbon radical having three to eight carbon atoms, which radicals can in turn be substituted by

a) a straight-chain or branched alkoxy radical having up to six carbon atoms or a straight-chain or branched alkenyloxy or alkynyloxy radical having three to six carbon atoms,

b) halogen, phenyl or an $\alpha$- or $\beta$-thienyl or $\alpha$- or $\beta$-furyl radical which in turn can be monosubstituted to trisubstituted in the nucleus by halogen, trifluoromethyl and/or alkyl or alkoxy each having 1–6 C atoms, or

c) a phenoxy radical, an $\alpha$- or $\beta$-thienyloxy radical or a cycloalkoxy radical having 3–7 carbon atoms, it being possible for the said radicals to be in turn monosubstituted to trisubstituted in the nucleus by halogen, trifluoromythyl and/or alkyl or alkoxy having 1–6 C atoms, and

$R^4$ and $R^5$ are each hydrogen which comprises

a) alkylating a compound of the formula XVII or XVIII

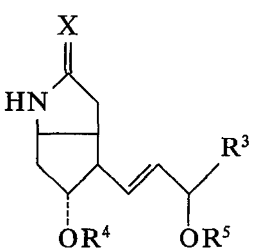

(XVII/XVIII)

wherein $R^3$ has the meaning given above, $R^4$ and $R^5$ are identical or different and represent a protective group which can readily be eliminated under neutral or basic conditions, and X is an oxygen or sulfur atom, with an alkyl halide of the formula IXX

Z – Y – Hal (IXX)

wherein Hal is iodine, chlorine or bromine and Y and Z have the meanings given above, or

b) reacting a compound of the formula XXII

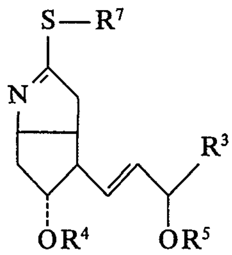

(XXII)

wherein $R^3$, $R^4$ and $R^5$ have the meanings given above and $R^7$ is a $C_{1-4}$-alkyl radical, with an amine of the formula

$$Z-Y-NH_2 \quad (XXIII)$$

wherein Z and Y have the meanings given above,

c) if appropriate, eliminating the protective groups $R^4$ and $R^5$ in a compound of the formula I by a hydrolysis with basic catalysis,

d) if appropriate, saponifying a compound of the formula I, in which Z is the radical $CO_2R^1$, $R^1$ representing the abovementioned radical and $R^4$ and $R^5$ being hydrogen, to give a compound of the formula I, in which Z represents the radical $CO_2R^1$, $R^1$ being hydrogen or a physiologically acceptable cation,

e) if appropriate, esterifying a compound of the formula I, in which Z represents the radical $CO_2R^1$, $R^1$ being hydrogen or a cation, to give a compound of the formula I, wherein Z represents the radical $CO_2R^1$, $R^1$ having the meanings mentioned under the formula I, but with the exclusion of hydrogen or a cation, and

f) if appropriate, in a compound of the formula I, in which Z represents the radical $CO_2R^1$, $R^1$ being a physiologically acceptable cation, exchanging this cation for another cation.

**Revendications pour les Etats contractants: BE, CH, DE, FR, GB, IT, LI, NL, SE**

1. Composés répondant à la formule:

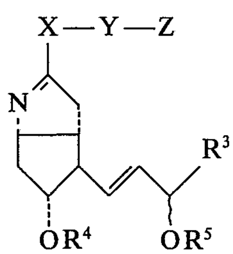

(I)

dans laquelle:

X représente un atome d'oxygène ou de soufre ou un groupe NH—;

Y représente un groupe alcoylène à chaîne droite ou ramifiée, ayant jusqu' à 8 atomes de carbone, ou un groupe aliphatique insaturé, à chaîne droite ou ramifiée, ayant de 3 à 8 atomes de carbone, ou un groupe cycloaliphatique ayant de 3 à 6 atomes de carbone ou un groupe phénylène;

Z représente un groupe de formule $-CO_2R^1$, $-CH_2OH$ ou $CH_2-N(R^2)_2$;

$R^1$ représentant l'hydrogène ou un groupe alcoyle à chaîne droite ou ramifiée, ayant jusqu' à 8 atomes de carbone ou un radical d'hydrocarbure aliphatique, insaturé, à chaîne droite ou ramifiée ayant de 3 à 6 atomes de carbone, ou un radical d'hydrocarbure cycloaliphatique ayant de 3 à 7 atomes de carbone ou un radical d'hydrocarbure araliphatique ayant de 7 à 9 atomes de carbone ou un ion métallique physiologiquement acceptable, $NH_4$ ou ammonium sous la forme du 5,5-diméthyl-dicyclohexyl- ou tris (hydroxyméthyl) méthylammonium,

$R^2$ représentant l'hydrogène ou un radical d'hydrocarbure aliphatique à chaîne droite ou ramifiée ayant jusqu' à 5 atomes de carbone ou bien $R^2$—$R^2$ ensemble représentent également un groupe $-(CH_2)_n-$ avec $n = 3$ à 6;

$R^3$ représente un groupe phényle non-substitué ou un groupe phényle, thiényle ou furyle qui peut être substitué de 1 à 3 fois dans le noyau par un halogène, un groupe trifluorométhyle, et/ou un groupe alcoyle ou alcoxy ayant chacun de 1 à 6 atomes de carbone, ou un groupe cycloaliphatique ayant de 3 à 8 atomes de carbone ou un groupe alcoyle à chaîne droite ou ramifiée ayant jusqu' à 8 atomes de carbone ou un radical d'hydrocarbure aliphatique insaturé, à chaîne droite ou ramifiée, ayant de 3 à 8 atomes de carbone, qui peuvent être substitués de leur côté par:

a) un groupe alcoxy à chaîne droite ou ramifiée ayant jusqu' à 6 atomes de carbone ou un groupe

alcényloxy ou alcynyloxy à chaîne droite ou ramifiée ayant de 3 à 6 atomes de carbone;

b) un halogène, un groupe phényle ou un groupe α- ou β-thiényle ou α- ou β-furyle qui de leur côté peuvent être substitués de 1 à 3 fois dans le noyau par un halogène, un groupe trifluorométhyle et/ou un groupe alcoyle ou alcoxy ayant de 1 à 6 atomes de carbone chacun;

c) un groupe phénoxy, un groupe α- ou β-thiényloxy, ou un groupe cycloalcoxy ayant de 3 à 7 atomes de carbone, les groupes mentionnés pouvant être de leur côté substitués de 1 à 3 fois dans le noyau par un halogène, un groupe trifluorométhyle et/ou un groupe alcoyle ou alcoyloxy ayant chacun de 1 à 6 atomes de carbone;

$R^4$, $R^5$ représentent chacun l'hydrogène.

2. Procédé de préparation de composés de formule I

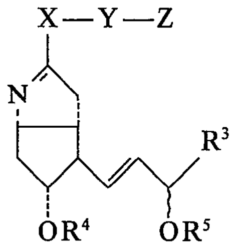

(I)

dans laquelle:

X représente un atome d'oxygène ou de soufre ou un groupe —NH—;

Y représente un groupe alcoylène à chaîne droite ou ramifiée, ayant jusqu' à 8 atomes de carbone, ou un groupe aliphatique insaturé, à chaîne droite ou ramifiée, ayant de 3 à 8 atomes de carbone, ou un groupe cycloaliphatique ayant de 3 à 6 atomes de carbone ou un groupe phénylène;

Z représente un groupe de formule $-CO_2R^1$, $-CH_2OH$ ou $-CH_2N(R^2)_2$;

$R^1$ représentant l'hydrogène ou un groupe alcoyle à chaîne droite ou ramifiée, ayant jusqu' à 8 atomes de carbone ou un radical d'hydrocarbure aliphatique, insaturé, à chaîne droite ou ramifiée ayant de 3 à 6 atomes de carbone, ou un radical d'hydrocarbure cycloaliphatique ayant de 3 à 7 atomes de carbone ou un radical d'hydrocarbure araliphatique ayant de 7 à 9 atomes de carbone ou un ion métallique physiologiquement acceptable, $NH_4$ ou un ion ammonium sous la forme du 5,5-diméthyl-dicyclohexyl-ou Tris (hydroxyméthyl)méthylammonium,

$R^2$ représentant l'hydrogène ou un radical d'hydrocarbure aliphatique à chaîne droite ou ramifiée ayant jusqu' à 5 atomes de carbone ou bien $R^2$—$R^2$ représentant ensemble un groupe $-(CH_2)_n-$, avec $n = 3$ à 6;

$R^3$ représentant un groupe phényle non-substitué ou un groupe phényle, thiényle ou furyle qui peut être de 1 à 3 fois substitué dans le noyau par un halogène, un groupe trifluorométhyle, et/ou un groupe alcoyle ou alcoxy ayant chacun de 1 à 6 atomes de carbone, ou un groupe cycloaliphatique ayant de 3 à 8 atomes de carbone ou un groupe alcoyle à chaîne droite ou ramifiée ayant jusqu' à 8 atomes de carbone ou un radical d'hydrocarbure aliphatique insaturé, à chaîne droite ou ramifiée, ayant de 3 à 8 atomes de carbone, qui peuvent être de leur côté substitués par:

a) un groupe alcoxy à chaîne droite ou ramifiée ayant jusqu' à 6 atomes de carbone ou un groupe alcényloxy ou alcynyloxy à chaîne droite ou ramifiée ayant de 3 à 6 atomes de carbone;

b) un halogène, un groupe phényle ou un groupe α- ou β-thiényle ou α- ou β-furyle qui peuvent de leur côté être substitués de 1 à 3 fois dans le noyau par un halogène, un groupe trifluorométhyle et/ou un groupe alcoyle ou alcoxy ayant de 1 à 6 atomes de carbone chacun;

c) un groupe phénoxy, un groupe α- ou β-thiényloxy, ou un groupe cycloalcoxy ayant de 3 à 7 atomes de carbone, les groupes mentionnés pouvant de leur côté être substitués de 1 à 3 fois dans le noyau avec un halogène, un groupe trifluorométhyle et/ou un groupe alcoyle ou alcoyloxy ayant de 1 à 6 atomes de carbone;

$R^4$, $R^5$ représentant chacun l'hydrogène,

ce procédé étant caractérisé en ce que:

a) on alcoyle un composé de formule XVII ou XVIII:

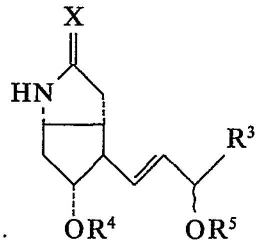

(XVII/XVIII)

dans laquelle $R^3$ a la signification ci-dessus, $R^4$ et $R^5$ sont identiques ou différents et représentent un groupe protecteur facilement éliminable dans des conditions neutres ou basiques et X représente un atome d'oxygène ou de soufre, avec un halogénure d'alcoyle de formule IXX

Z – Y – Hal (IXX)

dans laquelle Hal représente l'iode, le chlore ou le brome et Y et Z ont les significations indiquées ci-dessus, ou

b) on fait réagir un composé de formule XXII

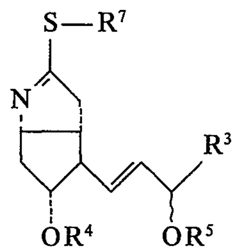

(XXII)

dans laquelle $R^3$, $R^4$ et $R^5$ ont les significations indiquées ci-dessus et $R^7$ représente un groupe alcoyle en $C_{1-4}$, avec une amine de formule:

$Z - Y - NH_2$ (XXIII)

dans laquelle Z et Y ont les significations indiquées ci-dessus,

c) éventuellement dans un composé de formule 1, on élimine les groupes protecteurs $R^4$ et $R^5$ par une hydrolyse catalysée par une base;

d) éventuellement on saponifie un composé de formule 1, dans lequel Z représente le groupe $CO_2R^1$, $R^1$ représentant le groupe mentionné ci-dessus et $R^4$ et $R^5$ représentant l'hydrogène en un composé de formule 1, dans lequel Z représente le groupe $CO_2R^1$, $R^1$ représentant l'hydrogène ou un cation physiologiquement acceptable;

e) éventuellement, on estérifie un composé de formule I, dans lequel Z représente le radical $CO_2R^1$, $R^1$ représentant l'hydrogène ou un cation, en un composé de formule I dans lequel Z représente le radical $CO_2R^1$, $R^1$ ayant la signification indiquée pour la formule I, mais ne représentant ni l'hydrogène ni un cation, et

f) éventuellement dans un composé de formule I dans lequel Z représente le groupe $CO_2R^1$, $R^1$ représentant un cation physiologiquement acceptable, on échange ce cation contre un autre cation.

3. Procédé de préparation de médicaments, caractérisé en ce qu'on met sous une forme appropriée pour l'administration thérapeutique un composé de formule I selon la revendication 1, éventuellement avec des véhicules et/ou des stabilisants pharmaceutiques usuels.

4. Médicament caractérisé en ce qu'il contient un composé de formule I selon la revendication 1, en mélange avec un véhicule et/ou un stabilisant pharmaceutique usuel.

5. Composé de formule I selon la revendication 1, pour utilisation comme médicament.

**Revendication pour l'Etat contractant: AT**

Procédé de préparation de composés de formule I

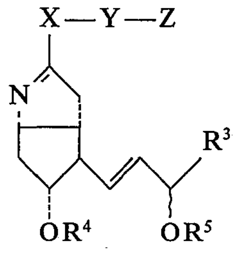

(I)

dans laquelle:

X représente un atome d'oxygène ou de soufre ou un groupe NH—;

Y représente un groupe alcoylène à chaîne droite ou ramifiée, ayant jusqu' à 8 atomes de carbone, ou un groupe aliphatique, insaturé, à chaîne droite ou ramifiée, ayant de 3 à 8 atomes de carbone, ou un groupe cycloaliphatique ayant de 3 à 6 atomes de carbone ou un groupe phénylène;

Z représente un groupe de formule $-CO_2R^1$, $-CH_2OH$ ou $CH_2N(R^2)_2$;

$R^1$ représentant l'hydrogène ou un groupe alcoyle à chaîne droite ou ramifiée, ayant jusqu' à 8 atomes de carbone ou un radical d'hydrocarbure aliphatique insaturé, à chaîne droite ou ramifiée ayant de 3 à 6 atomes de carbone, ou un radical d'hydrocarbure cycloaliphatique ayant de 3 à 7 atomes de carbone ou un radical d'hydrocarbure araliphatique ayant de 7 à 9 atomes de carbone ou un ion métallique physiologiquement acceptable, $NH_4$ ou ammonium sous la forme du 5,5-diméthyl-dicyclohexyl- ou Tris (hydroxyméthyl)-méthyl ammonium;

$R^2$ représentant l'hydrogène ou un radical d'hydrocarbure aliphatique à chaîne droite ou ramifiée ayant jusqu' à 5 atomes de carbone ou bien $R^2$—$R^2$ ensemble représentant également un groupe $-(CH_2)_n-$, avec n = 3 à 6;

$R^3$ représente un groupe phényle non-substitué ou un groupe phényle, thiényle ou furyle qui peut être substitué de 1 à 3 fois dans le noyau par un halogène, un groupe trifluorométhyle, et/ou un groupe alcoyle ou alcoxy ayant chacun de 1 à 6 atomes de carbone, ou un groupe cycloaliphatique ayant de 3 à 8 atomes de carbone, ou un groupe alcoyle à chaîne droite ou ramifiée ayant jusqu' à 8 atomes de carbone ou un radical d'hydrocarbure aliphatique, insaturé, à chaîne droite ou ramifiée, ayant de 3 à 8 atomes de carbone, qui peuvent être de leur côté substitués par:

a) un groupe alcoxy à chaîne droite ou ramifiée ayant jusqu' à 6 atomes de carbone ou un groupe alcényloxy ou alcynyloxy à chaîne droite ou ramifiée ayant de 3 à 6 atomes de carbone;

b) un halogène, un groupe phényle ou un groupe $\alpha$- ou $\beta$-thiényle ou $\alpha$- ou $\beta$-furyle qui de leur côté peuvent être substitués de 1 à 3 fois dans le noyau par un halogène, un groupe trifluorométhyle et/ou un groupe alcoyle ou alcoxy ayant chacun de 1 à 6 atomes de carbone;

c) un groupe phénoxy, un groupe $\alpha$- ou $\beta$-thiényloxy ou un groupe cycloalcoxy ayant de 3 à 7 atomes de carbone, les groupes mentionnés pouvant de leur côté être substitués de 1 à 3 fois dans le noyau par un halogène, un groupe trifluorométhyle et/ou un groupe alcoyle ou alcoyloxy ayant chacun de 1 à 6 atomes de carbone

$R^4$, $R^5$ représentent chacun l'hydrogène,

ce procédé étant caractérisé en ce que:

a) on alcoyle un composé de formule XVII ou XVIII

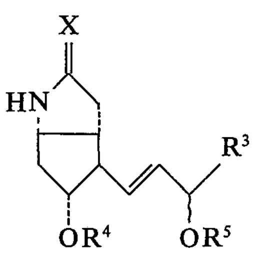

(XVII/XVIII)

dans laquelle $R^3$ a la signification indiquée ci-dessus, $R^4$ et $R^5$ sont identiques ou différents et représentent un groupe protecteur facilement éliminable dans des conditions neutres ou basiques et X représente un atome d'oxygène ou de soufre, avec un halogénure d'alcoyle de formule IXX

Z – Y – Hal (IXX)

dans laquelle Hal représente l'iode, le chlore ou le brome, et Y et Z ont les significations indiquées ci-dessus, ou

b) on fait réagir un composé de formule XXII

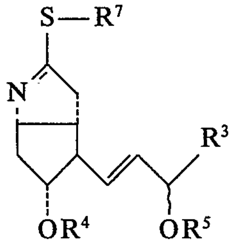

(XXII)

dans laquelle $R^3$, $R^4$ et $R^5$ ont les significations indiquées ci-dessus et $R^7$ représente un groupe alcoyle en $C_{1-4}$, avec une amine de formule:

$$Z - Y - NH_2 \qquad (XXIII)$$

dans laquelle Z et Y ont les significations indiquées ci-dessus;

c) éventuellement dans un composé de formule I, on élimine les groupes protecteurs $R^4$ et $R^5$ par une hydrolyse catalysée par une base;

d) éventuellement on saponifie un composé de formule I, dans lequel Z représente le groupe $CO_2R^1$, $R^1$ représentent le groupe mentionné ci-dessus, et $R^4$ et $R^5$ représentant l'hydrogène, en un composé de formule I dans lequel Z représente le groupe $-CO_2R^1$, $R^1$ représentant l'hydrogène ou un cation physiologiquement acceptable;

e) éventuellement, on estérifie un composé de formule I, dans lequel Z représente le groupe $CO_2R^1$, $R^1$ représentant l'hydrogène ou un cation, en un composé de formule I dans lequel Z représente le groupe $CO_2R^1$, $R^1$ ayant la signification indiquée pour la formule I, mais ne représentant ni l'hydrogène, ni un cation, et

f) éventuellement dans un composé de formule I, dans lequel Z représente le groupe $CO_2R^1$; $R^1$ représentant un cation physiologiquement acceptable, on échange ce cation contre un autre cation.